(19) Europäisches Patentamt

European Patent Office

Office européen des brevets

(11) **EP 0 711 148 B1**

(12) **EUROPEAN PATENT SPECIFICATION**

(45) Date of publication and mention
of the grant of the patent:
**30.08.2000 Bulletin 2000/35**

(51) Int. Cl.7: **A61K 9/127**

(21) Application number: **94922214.5**

(86) International application number:
**PCT/CA94/00409**

(22) Date of filing: **28.07.1994**

(87) International publication number:
**WO 95/03787 (09.02.1995 Gazette 1995/07)**

(54) **BIPHASIC MULTILAMELLAR LIPID VESICLES**

ZWEIPHASIGE MULTILAMELLARE LIPIDVESIKEL

VESICULES LIPIDIQUES MULTILAMELLAIRES BIPHASIQUES

(84) Designated Contracting States:
**AT BE CH DE DK ES FR GB GR IE IT LI LU MC NL
PT SE**
Designated Extension States:
**LT SI**

(30) Priority: **28.07.1993 US 98102**

(43) Date of publication of application:
**15.05.1996 Bulletin 1996/20**

(73) Proprietor:
**PHARMADERM LABORATORIES LTD.
Saskatoon, Saskatchewan S7N 4L8 (CA)**

(72) Inventor: **FOLDVARI, Marianna
Saskatoon, Saskatchewan S7J 4J6 (CA)**

(74) Representative:
**VOSSIUS & PARTNER
Postfach 86 07 67
81634 München (DE)**

(56) References cited:
**EP-A- 0 130 577          WO-A-90/12565
WO-A-91/01719          WO-A-92/06676
WO-A-93/00894**

**Description**

Liposomes are microscopic vesicles composed of concentric or single phospholipid bilayers which enclose aqueous phases. These vesicles can serve as drug carriers for hydrophobic as well as hydrophilic substances, including various drug molecules and peptides. Liposomes are prepared mainly from phospholipids of different headgroups and chain lengths, and cholesterol. Due to physicochemical similarities with cellular membranes, liposomes are biocompatible, biodegradable and non-toxic vehicles for drug molecules.

Liposomes are prime candidates for the systemic as well as topical delivery of drugs. Several studies showed that liposome encapsulation advantageously alters the pharmacokinetic fate of the drug after topical application. The first experimental evidence that liposomes may penetrate into the skin and deposit within the dermis was obtained recently by using a novel small particle-size electrondense marker (colloidal iron), which can be efficiently encapsulated into liposomes of various sizes and compositions, and can be easily identified in cells and tissues by electron microscope.

However, current liposome technology suffers from a number of important drawbacks that has prevented its widespread use in dermal applications and in systemic delivery. For example, liposome formulations present consistency problems for skin applications that have so far been solved by either adding viscosity-increasing agents such as cellulose derivatives to the final liposome preparation or by using higher concentrations of phospholipids in the preparation of the liposomes.

The addition of viscosity-increasing agents to the final liposome preparation usually dilutes the product but more importantly, these agents were shown to decrease the release of encapsulated drug from the liposomes. Also, viscosity-increasing agents have a detrimental effect on the integrity of the liposomes. With regard to phospholipids, they are the main and also the most expensive liposome-forming ingredient. Their use as a viscosity-increasing agent is therefore not economically practical.

Another problem with current liposome technology is the available methods for making them. Methods such as the solvent evaporation method are very difficult to use on a large scale. Furthermore, the solvent evaporation method requires the use of toxic organic solvents such as chloroform for the dissolution of liposome-forming components. Ultimately, some of these toxic solvents are found in the final product. Also, not all biologically active substances are soluble in these organic solvents.

Impotence, the partial or complete inability of the male to perform the sexual act or to achieve orgasm, may be psychogenic and/or organic in nature. Initially, psychogenic etiology was considered to account for 95 percent of cases of impotence but it is now apparent that organic factors are almost invariably involved in cases of impotence. Thus, impotence is best characterized as either of predominantly organic or predominantly psychogenic etiology.

Impotence is a socially crippling disease. It detracts from the patient's self esteem and in some cases his ability to interact with others. Current therapy modes include psychotherapy, microsurgery (to restore compromised vascular supply), implantation of penile prosthesis and pharmacotherapy.

Numerous agents have been employed in the therapy of impotence. Hormonal manipulation, such as replacement androgen therapy, has been used. However, patients with organic impotence are rarely candidates for hormonal manipulation. Thus, this mode of therapy should be strictly limited to cases in which endocrine deficiencies are established. Furthermore, in about 50 percent of patients with an endocrinopathy, a psychogenic etiology can be shown to be the predominant factor in erectile difficulties. Therefore, in the vast majority of impotent men, vascular and neurologic factors are the underlying causes. The most commonly used treatment for these patients is the implantation of penile prostheses. The invasive nature of this technique, coupled with the increasing evidence of mechanical failure, surgical complications, or infection has again focused attention on the development of pharmacological agents with a potential for improving the libido and quality of erections. Several agents have been tested e.g. bromocriptine, glyceryl trinitrate, zinc, oxytocin, yohimbine and nitroglycerin. Intracorporeal injection of vasoactive agents is now considered the treatment of choice for many patients with organic impotence (Kattan et. al., 1991).

Injection of papaverine, a smooth muscle relaxant, or a combination of papaverine and phentolamine (an a-adrenergic blocker) directly into the corpus cavernosum (either of the two erectile columns of the dorsum of the penis) has been shown to be an effective therapy in impotence. Recently, intercavernosal injections of prostaglandin $E_1$ (alprostadil), a naturally occurring chemical derived from dihomo-gammalinolenic acid (20:3∞6) was discovered to also induce erection.

Injection of prostaglandin $E_1$ results in vasodilation, with increased arterial inflow and decreased venous outflow by occlusion of draining venules, probably through relaxation of corporal smooth muscle. Due to its potent relaxant effect on vascular smooth muscle, prostaglandin $E_1$ is used to maintain the patency of the ductus arteriosus in the neonate. This is the only currently approved (FDA) indication for prostaglandin $E_1$. The effect of prostaglandin $E_1$ on erectile dysfunction is known to be dose dependent.

Prostaglandin $E_1$ ($PGE_1$), a potent relaxant of the vascular smooth muscle, has been shown to be effective and safe in the treatment of impotence by increasing arterial inflow through vasodilatation and decreasing venous outflow by the occlusion of draining venules due to relaxation of corporal smooth muscle. As opposed to other previously used drugs such as papaverine or phentolamine, $PGE_1$ is not as frequently associated with the common side effects e.g. priapism, plaques at the injection site and liver function abnormalities, therefore it is clinically more acceptable. $PGE_1$ is usually self-injected into the corpus cavernosum through the lateral aspect of the shaft of the penis. This type of administration, however, is associated with penile discomfort, pain at the injection site and the inconvenience of application prior to intercourse. Topical application of $PGE_1$ would be in ideal route of administration, however, the drug itself (without a delivery system) cannot penetrate the skin in adequate concentration and would be metabolized within the skin very quickly before reaching the underlying tissues.

It is estimated that condylomata acuminata contributes to 1.3-1.4 million office visits per year in the United States, representing a 580% increase between 1966 and 1983 (Center for Disease Control, 1983). Condylomata acuminata is caused by the human papillomavirus (HPV) and it is one of the most commonly diagnosed viral sexually transmitted diseases. More than 50 types of HPV has been categorized and new types are constantly being identified. Certain subsets of HPV (mostly type 16 and 18) has been shown to be associated with malignant tumors of the anogenital tract, respiratory tract and the skin.

Conventional therapy for genital warts has included podophyllin, cryotherapy, trichloroacetic acid, electrical cautery, laser ablation and conventional surgery, and has been directed primarily at visible lesions. Recurrences after therapy for HPV infections is very common, which could be due to incomplete eradication of the virus since the presence of residual virus in normal-appearing tissue has been demonstrated.

Interferon has activity against papillomaviruses, and cure infected cells by eliminating extrachromosomal viral DNA. Systemic application (i.m. injection) of interferon alpha in patients with genital warts was shown to be fairly successful, however it is associated with various side effects such as fever, myalgias, headache, nausea, fatigue. Intralesional IFN treatment appears to be a more promising approach for visible lesions, but it is not suitable for latent or subclinical infections. Initially highly positive results (90% complete response) were reported with topical natural leukocyte IFN. Vesterinen et al. reported colposcopic remission in five out of eight patients with vaginal flat condylomas treated with a potent topical IFN cream. However, in spite of the improvement of clinical appearance, the cytology remained positive in all cases and two responding patients had recurrences in two months.

PCT International Application WO 92/06676 discloses a "cold-loading" technique for filling the amorphous central cavity of paucilamellar lipid vesicles with water-immiscible material. EPA 0,130,577 discloses a method of forming liposomes by mixing liposome membrane components with a water-soluble non-volatile solvent and then dispersing the mixture in an aqueous medium. There is no mention of encapsulating an emulsion in the liposomes. PCT International Applications WO 93/00894 and WO 91/01719 relate to use of $PGE_2$ for the treatment of erectile dysfunction and to use of interferon alpha for the treatment of human papillomavirus genital warts, respectively. Neither reference teaches use of a multilamellar lipid vesicle having an emulsion in its central core compartment as a vehicle for administration of the pharmaceutically active agent. PCT International Application WO 90/12565 relates to a method of preparing water-containing formulations of phospholipids in the presence of swelling accelerators, and the use of the gels so formed for the preparation of liposomal formulations by dilution with water. Encapsulation of emulsions in the liposomes is not taught.

SUMMARY OF THE INVENTION

The present invention relates to a multilamellar lipid vesicle, particularly to biphasic multilamellar lipid vesicles. The biphasic lipid vesicle comprises a plurality of spaced apart lipid bilayers comprising a liposome-forming component and optionally a biologically active agent (for the purpose of this specification "biologically active agent or compound" shall mean any agent that has a pharmacological, pharmaceutical or cosmetic effect) entrapped within the bilayers. The lipid vesicle also comprises peripheral aqueous solution compartments formed between the lipid bilayers and a central lipophilic core compartment substantially at the center of the multilamellar lipid vesicle.

Preferably, the lipid bilayers are formed from an anhydrous proliposome gel comprising the liposome-forming component in admixture with a pharmaceutically acceptable hydrophilic solvent other than water. The liposome-forming component is usually selected from the group consisting of glycolipids, phospholipids, ceramides and mixtures thereof and optionally, a fatty substance such as cholesterol is added to enhance the strength of the lipid bilayers.

Optionally, the lipid bilayers can further comprise at least one cutaneous or percutaneous penetration enhancer agent entrapped within the lipid bilayers or absorbed on the surface thereof. Preferred penetration enhancer agents include fatty acylated amino acids such as monolauroyllysine.

Incorporation of lipophilic substances in the central core compartment of the multilamellar lipid vesicle is accomplished through a lipophilic substance-in-water emulsion in the form of a droplet which comprises on its surface a liposome-formation promoting agent adapted to stabilize the liposome-forming component and to promote formation of lipid vesicles around the droplet. The liposome-formation promoting agent is preferably a surfactant or a primary cationic emulsifier such as linoleamidopropyl PG-Dimonium chloride phosphate (PEFA), cocamidopropyl PG-dimonium chloride phosphate or stearamido PG-dimonium chloride phosphate.

Hence, oil droplets or solid/semisolid lipophilic ingredients can be encapsulated into liposomes. This encapsulation specifically includes coating the oil droplets or solid/semisolid lipophilic ingredients with a suitable surfactant followed by a reduction of the size of the droplets to below about 0.5 μM using a high pressure homogenizer. This procedure provides a milky solution which can be used in place of an aqueous phase in the formation of liposomes. Coated droplets are encapsulated into the central core compartment of the liposomes and are then surrounded by multiple bilayers. In this type of system, the end product can contain the targeted encapsulated drug in three different compartments within the liposome: lipid bilayers, the peripheral aqueous solution compartments and the central lipophilic core.

Preferred lipophilic substances that can be incorporated in the central core of the multilamellar lipid vesicles of the present invention include oils and solid or semisolid liphophilic consistency enhancers in which biologically active lipophilic compounds can be incorporated.

Also within the scope of the present invention is a liposome composition comprising a population of biphasic multilamellar lipid vesicles, as described above.

Also within the scope of the present invention is a process for the preparation of biphasic multilamellar lipid vesicles having a plurality of lipid bilayers. The process comprises forming a lipid phase melt by mixing a liposome-forming component and preferably a fatty substance adapted to enhance the strength of lipid bilayers with a pharmaceutically acceptable hydrophilic solvent other than water and optionally a biologically active compound to produce an anhydrous proliposome gel in the form of a stable liposome-forming lipid phase melt. An lipophilic solution optionally comprising a biologically active compound is then added to the proliposome gel and the multilamellar lipid vesicles are recovered.

Preferably, the lipophilic solution is a lipophilic substance-in-water emulsion in the form of a plurality of droplets comprising on their surface a liposome-formation promoting agent adapted to stabilize the liposome-forming component and to promote the formation of lipid vesicles around the droplets. This solution is prepared by dissolving liposome-formation promoting agent in water and adding the lipophilic substance to the water. The lipophilic substance preferably comprises at least one component selected from the group consisting of an oil and a solid or semisolid lipophilic consistency enhancer.

Also within the scope of the present invention is a multilamellar lipid vesicle to be used particularly for topical applications. The lipid vesicle comprises a plurality of spaced apart lipid bilayers comprising a liposome-forming component preferably in admixture with a fatty substance adapted to enhance the strength of the bilayers and optionally a biologically active agent entrapped within the bilayers. The lipid vesicle also comprises peripheral aqueous solution compartments formed between the lipid bilayers and a central aqueous core compartment substantially at the center of the multilamellar lipid vesicle. The peripheral compartments comprise at least traces of a pharmaceutically acceptable hydrophilic solvent other than water. The multilamellar lipid vesicles are devoid of traces of organic solvents, such as chloroform, which are usually employed in conventional liposome-forming techniques for dissolving the liposome-forming component.

Preferably, the lipid bilayers are formed from an anhydrous proliposome gel comprising the liposome-forming component in admixture with the fatty acid substance and a pharmaceutically acceptable hydrophilic solvent other than water. The liposome-forming component is usually selected from the group consisting of glycolipids, ceramides, phospholipids and mixtures thereof and the fatty substance is usually cholesterol.

Optionally, the lipid bilayers can further comprise at least one cutaneous or percutaneous penetration enhancer agent entrapped within the lipid bilayers or absorbed on the surface thereof. Preferred penetration enhancer agents include fatty acylated amino acids such as monolauroyllysine.

Two of the multiple applications of the multilamellar lipid vesicles of the present invention reside in their use for the treatment of erectile dysfunctions and papilloma-virus infections. For the treatment of erectile dysfunctions, a prostaglandin, preferably PGE$_1$, is incorporated in the lipid bilayers. As for the treatment of papillomavirus infections, interferon alpha is incorporated in the lipid bilayers.

Also within the scope of the present invention is a liposome composition comprising a population of multilamellar lipid vesicles, as described above. The multilamellar lipid vesicle population has a substantially uniform size distribution, preferably ranging between 0.1 and 10 μm, while being substantially devoid of aggregated or fused liposomes, and devoid of traces of organic solvents usually employed for dissolving lipid-forming components.

Also within the scope of the present invention is a process for the preparation of multilamellar lipid vesicles having a plurality of lipid bilayers. The process comprises forming a lipid phase melt by mixing a liposome-forming component preferably with a fatty substance adapted to enhance the strength of lipid bilayers, a pharmaceutically acceptable hydrophilic solvent other than water and optionally a biologically active compound to produce an anhydrous proliposome gel in the form of a stable liposome-forming lipid phase melt. An aqueous solution optionally comprising a biologically active compound is then added to the proliposome gel and the multilamellar lipid vesicles are recovered.

Also within the scope of the present invention is a composition for preparing multilamellar lipid vesicles having a plurality of lipid bilayers. The composition comprises a liposome-forming component, preferably in admixture with a fatty acid substance adapted to enhance the strength of the lipid bilayers, and a pharmaceutically acceptable hydrophilic solvent other than water to produce an anhydrous proliposome gel in the form of a stable liposome-forming lipid phase melt.

Also within the scope of the invention is a method for the treatment of erectile dysfunctions. This method comprises topically administering to a patient in need thereof an effective amount of a liposome composition comprising a population of multilamellar lipid vesicles. The multilamellar lipid vesicles comprise a plurality of spaced apart lipid bilayers comprising a liposome-forming component, preferably in admixture with a fatty substance adapted to enhance the strength of the lipid bilayers, and $PGE_1$ entrapped within the lipid bilayers. The multilamellar lipid vesicles also comprise peripheral aqueous solution compartments formed between the lipid bilayers and a central aqueous or lipophilic core compartment substantially at the center of the multilamellar lipid vesicle. The peripheral compartment comprise at least traces of a pharmaceutically acceptable hydrophilic solvent other than water. The multilamellar lipid vesicle population has a substantially uniform size distribution while being substantially devoid of aggregated or fused liposomes and devoid of traces of organic solvents suitable for dissolving the lipid bilayers, for the purpose of treating erectile dysfunctions.

Also within the scope of the present invention is the use of a topical liposome composition for the application of $PGE_1$ in the treatment of erectile dysfunctions. The liposome composition comprises the multilamellar lipid vesicles or the biphasic multilamellar lipid vesicles described above. Also within the scope of the present invention is the use of the multilamellar lipid vesicles or the biphasic multilamellar lipid vesicles described above in the preparation of a topical medicament comprising $PGE_1$ for the treatment of erectile dysfunctions. The invention also relates to a method for the treatment of papillomavirus infections. The method comprises topically administering to a patient in need thereof an effective amount of a liposome composition comprising a population of multilamellar lipid vesicles. The multilamellar lipid vesicles comprises a plurality of spaced apart lipid bilayers comprising a liposome-forming component, preferably in admixture with a fatty substance adapted to enhance the strength of the lipid bilayers, and interferon alpha entrapped within the lipid bilayers. The multilamellar lipid vesicles comprise peripheral aqueous solution compartments formed between the lipid bilayers and a central aqueous or lipophilic core compartment substantially at the center of the multilamellar lipid vesicle. The peripheral compartments comprise at least traces of a pharmaceutically acceptable hydrophilic solvent other than water. The multilamellar lipid vesicle population has a substantially uniform size distribution while being substantially devoid of aggregated or fused liposomes and devoid of traces of organic solvents suitable for dissolving said lipid bilayers.

Also within the scope of the present invention is the use of a topical liposome composition for the application of $PGE_1$ in the treatment of impotence. The liposome composition comprises a population of multilamellar lipid vesicles of biphasic multilamellar lipid vesicles.

Also within the scope of the present invention is the use of multilamellar lipid vesicles or biphasic multilamellar lipid vesicles in the preparation of a medicament for the treatment of papillomavirus infections.

The multilamellar lipid vesicles developed in the context of the present invention have shown high encapsulation efficiency of hydrophilic, lipophilic and oil-like active ingredients. The simplicity of the method by which the vesicles are prepared allows production on a large scale. With the method of the present invention, a finished product which is not a liposomal intermediate can be produced and then incorporated into another cream-based or gel matrix to make a final product.

The term biologically active agent" when used herein is intended to designate compounds having a medicinal activity as well as compounds used in the preparation of cosmetics.

One of the decisive advantages of the method developed in the context of the present invention is the fact that it does not make use of toxic organic solvents such as chloroform or methanol. In fact, one of the important features of the present invention is the use of liposome-forming components such as phospholipids and other necessary excipients in the form of a "melt" system which is prepared using a pharmaceutically acceptable hydrophilic solvent such as propylene glycol. Propylene glycol is used as a plasticizer and an aid to prepare a uniform melt. The resulting liposomes are thus free of any trace of organic solvents.

With the use of the process of the present invention, a more physically stable liposome composition is obtained. Hence, a more uniform liposome-size distribution is achieved while aggregation and fusion of liposomes is reduced to a minimum. Furthermore, the consistency of the final liposome composition is also substantially improved. It has also been demonstrated that encapsulation efficiency is higher or at least as good as the more conventional solvent evaporation method. It would also appear that encapsulation is more reproducible and this is indicated by smaller standard deviations.

Other important features of the liposomal composition of the present invention include the possibility of encapsulation and coencapsulation of a wide range of active ingredients with different physico-chemical properties. For example, lipid compounds which are not soluble in organic solvents can now be used for liposome construction. This type of product could not even be made with the traditional solvent evaporation methods which involve the dissolution of liposome ingredients in a mixture of chloroform and methanol.

Furthermore, the multilamellar lipid vesicles developed using the process of the present invention can be used to deliver oil droplets containing solubilized lipophilic drugs or oily plant extracts. The present invention also allows the encapsulation of lipophilic solid/semisolid consistency enhancers into the central core compartment of the multilamellar lipid vesicles. This provides enhanced viscosity for the final product and eliminates the requirements to add viscosity-increasing agents to the final liposome preparation.

Also, the formulation of liposomal IFN has contributed to the development of an effective form of topical interferon, and offer the advantage of treating latent HPV infections as well as visible genital warts and could be administered by the patients at home without the need for multiple painful local or i.m. injections.

The present invention will be more readily illustrated by referring to the following description.

## IN THE DRAWINGS

Figure 1a is a photograph of multilamellar liposomes prepared with solvent evaporation method.
Figure 1b is a photograph of multilamellar liposomes prepared by the present inventor using "anhydrous plastic proliposome-gel" ('melt') method.
Figure 2 is a schematic side sectional view of a MLV with a central aqueous core.
Figure 3 is a schematic side sectional view of a MLV with a central lipophilic core comprising an oil droplet.
Figure 4 is a schematic side sectional view of a MLV with a central lipophilic core comprising a semisolid consistency enhancer.

## DETAILED DESCRIPTION OF THE INVENTION

The invention relates to liposomal compositions for oral and topical use. The compositions of the present invention are particularly suitable for the dermal and transdermal delivery of biologically active compounds such as prostaglandins and proteins. The liposome compositions of the present invention can be used for the topical administration of biologically active compounds to hairy or hairless areas of the skin. They can also be used in mucus membranes for nasal, buccal, ocular, otic, vaginal and urethral administration. The liposome compositions can also be used for localized (intradermal and intramucosal) or systemic (transdermal or transmucosal) delivery as well as in subcutaneous or intracutaneous injection for slow release depot in or beneath the skin.

Each aspect of the present invention will be described in detail and followed by specific examples of applications in the treatment of erectile dysfunction and papillomavirus infections. The first section will discuss the preparation of multilamellar lipid vesicles with a central aqueous core. This will be followed by a second section describing the preparation of the biphasic multilamellar lipid vesicles having incorporated therein a central lipophilic core.

### A. Preparation of multilamellar vesicles with a central aqueous core compartment

#### 1° Formation of an anhydrous plastic proliposome gel

The anhydrous proliposome gel is an important intermediate in the formation of the multilamellar vesicles of the present invention. This gel is prepared using a mixing technique that is used frequently in emulsion technology.

A liposome-forming component and other necessary excipients are melted with a pharmaceutically acceptable hydrophilic solvent such as propylene glycol which would normally be part of the formula's aqueous phase. In addition, the following (or other similar) extracts can be used in place of the propylene glycol for mixing with the liposome forming component to produce an anhydrous proliposome gel: lavender, sage, rosemary, roman chamomile, carrot, myrrh, pot marigold, elder, arnica, eucalyptus, witch hazel, devil's claw, meadow sweet, willow, silver birch, rose hips, German chamomile, horse chestnut, cypress, incense, benjamine, thyme, gingko biloba and yarrow.

The term "liposome-forming component", when used herein, is intended to designate any substance that is used as a major component in the preparation of lipid bilayers. Typical liposome-forming components include glycolipids, lecithins, phospholipids, ceramides or mixtures thereof which are used as a primary ingredient in the formation of the lipid bilayer. However, other natural and synthetic compounds having the required amphipatic character can be used to form liposomes. The choice of the appropriate materials is within the knowledge of the person skilled in the art. Examples include phosphatidylethanolamine, lysolecithin, lysophosphatidylethanolamine, phosphatidylserine, phosphatidylinositol, sphingomyelin, cardiolipin, phosphatidic acid and the cerebrosides, ether lipids and phytanols.

The liposomal formulations of the present invention preferably contain saturated and/or unsaturated phospholipids, more preferably phosphatidylcholine, lysophosphatidylcholine, phosphatidylserine, phosphatidylethanolamine, glycolipids and ceramides. The phospholipids are preferably in combination with a penetration enhancing agent such as monolauroyllysine or methyl salicylate to achieve predominantly transdermal delivery potential.

With regard to the term "fatty substance", it is intended to designate any substance that is used as a secondary agent to form the lipid bilayers. This secondary agent is used to enhance the strength of the lipid bilayers formed from the appropriate liposome-forming components. Examples of useful fatty substances include steroids such as cholesterol, corprostanol, cholestanol and cholestane and saturated long chain fatty acids such as stearic acid to improve physical stability and appearance of the product.

As for the biologically active agent that can be entrapped within the peripheral aqueous solution compartments or within the lipid bilayers, such compound includes, but is not limited to, nucleic acids, polynucleotides, anti-bacterial compounds, antiviral compounds, antifungal compounds, anti-parasitic compounds, tumoricidal compounds, proteins, toxins, enzymes, hormones, neurotransmitters, glycoproteins, immunoglobulins, immunomodulators, dyes, radiolabels, radio-opaque compounds, fluorescent compounds, polysaccharides, cell receptor binding molecules, anti-inflammatories, antiglaucomic agents, mydriatic compounds, local anesthetic, nucleoside analogues, etc.

The hydrophilic solvent is used as a plasticizer of the liposome-forming component and an aid to prepare a uniform melt. The hydrophilic solvent is preferably non-toxic. Examples of hydrophilic solvents include but are not restricted to propylene glycol, glycerol, polyethylene glycol having a molecular weight ranging between 300 and 8000 and ethanol. The resulting melt can be described as being an anhydrous plastic proliposome gel. This anhydrous plastic proliposome gel contains all the lipid phase ingredients and can be prepared and stored in advance in large quantities. It is a semisolid material with a homogenous consistency.

2° Formation of the multilamellar lipid vesicles

Once the anhydrous plastic proliposome gel has been formed, the hydrophilic ingredients which are to form the liposomal composition, such as penetration enhancers, preservatives and the like, are prepared separately as an aqueous solution which is added to the lipid phase melt previously heated to the appropriate melting temperature that can range from 40°C to 80°C and vigorously mixed by any given technique which allows the achievement of the desired product size. Examples of mixing techniques include vortexing or propeller mixing. At this stage, it is also possible to incorporate solid biologically active agents that will be entrapped within the lipid bilayers.

This procedure is suitable for the preparation of various amounts of topical liposomal product. If vortex mixing is used as the agitation, up to about 20 g of the product can be prepared. If a laboratory scale propeller mixer is used, up to about 2000 g of the product can be made. This formulation procedure can also be adapted for large scale manufacturing. Hence, the propeller mixing technique can be directly scaled up by geometrically increasing the size of the vessel and the diameter of the propeller mixer. However, as the vessel size increases, the preferred set up would be a combination mixer i.e. a high intensity mixer with propeller mixer and a scraped surface agitator. The aqueous phase is pumped from tank A to tank B containing the anhydrous plastic proliposome gel at the required temperature and mixed. This procedure is suitable for the production of any topical liposomal product on a large scale.

Liposomal compositions can be prepared with the multilamellar lipid vesicles of the present invention by using appropriate pharmaceutical additives. For example, it might be required to add viscosity increasing agents to the final liposome preparation. The addition of other pharmaceutically acceptable compounds is within the purview of the person skilled in the art.

3° Characteristics of the final multilamellar lipid vesicle product

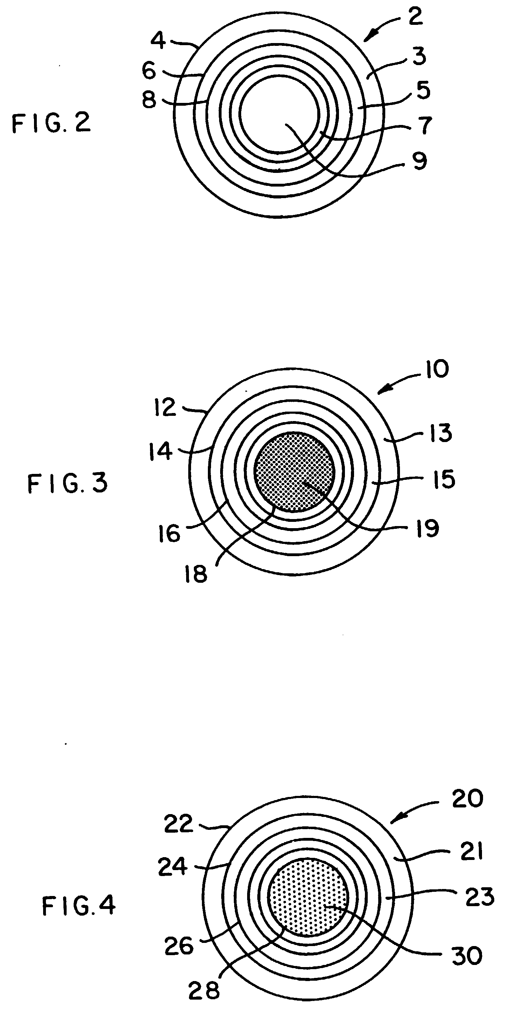

A schematic representation of a multilamellar lipid vesicle prepared in accordance with the process described above is shown at Figure 2. The multilamellar lipid vesicle, generally designated by reference numeral 2, is made of a series of spaced apart lipid bilayers 4, 6 and 8 which define a series of peripheral aqueous solution compartments 3 and 5. The smallest lipid bilayer 7 defines in its center a central aqueous core compartment 9.

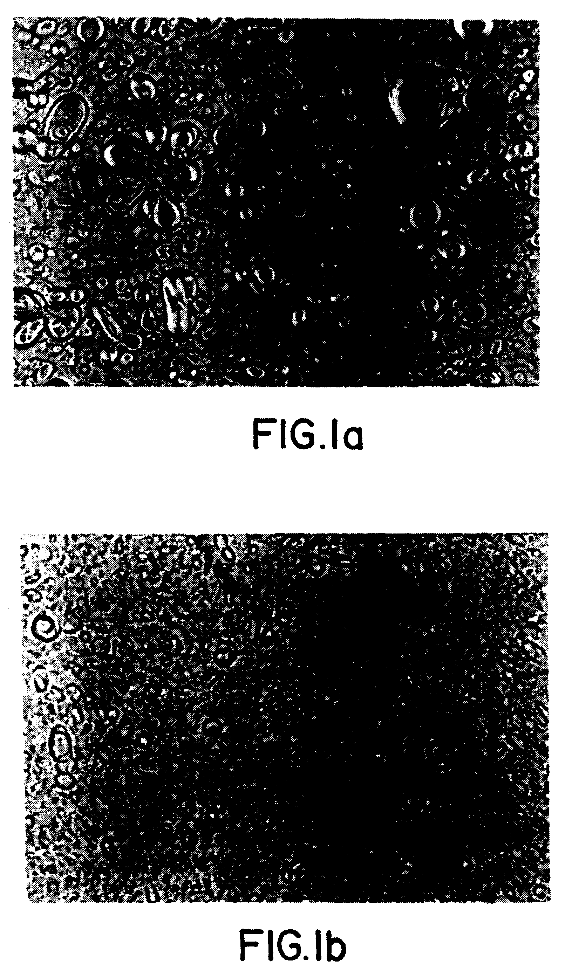

In order to demonstrate the difference in properties observed in the liposome population produced in accordance with the method of the present invention, comparative tests were conducted between two liposome compositions prepared from the same ingredients but using either the solvent evaporation method or the anhydrous plastic proliposome gel method of the present invention. Figure 1a is a photograph of the liposome population prepared using the solvent evaporation method and Figure 1b is a photograph of the liposome population prepared using the anhydrous plastic proliposome gel method. As it can be seen by comparing Figures 1a and 1b, the liposome population obtained using the anhydrous plastic proliposome gel method has a liposome size distribution which is substantially more uniform than the liposome population obtained using the solvent evaporation method. Also, minimal amounts of aggregated or fused liposomes are formed when using the anhydrous plastic proliposome gel method whereas large aggregates can be observed in the liposome population obtained using the solvent evaporation method. Table 1 summarizes the physical properties of the liposome formulations shown in Figures 1a and 1b.

TABLE 1

| Comparison of two identical formulas prepared by: a) solvent evaporation method (Formula #1) b) "anhydrous plastic proliposome-gel" ('melt') method (Formula #2) | | |
|---|---|---|
| **Formula no.** | **Organoleptic properties** | **Microscopyl/liposome size distribution** |
| 1 (Fig. 1a) | white cream, homogeneous | MLV, 1-30μm, aggregation, fusion |
| 2 (Fig. 1b) | white cream, homogeneous | MLV, 1-10μm, even distribution of liposomes, no fusion or aggregation |

Also, it has been demonstrated that the encapsulation efficiency of proteins into liposomes is higher or at least as good as the encapsulation efficiency using the solvent evaporation method without the drawback of traces of toxic solvents such as chloroform that are observed with the solvent evaporation method. Furthermore, it is important to mention that encapsulation seems to be more reproducible when using the anhydrous plastic proliposome gel method which presents a much smaller standard deviation than the solvent evaporation method. The results are summarized in Table 2.

TABLE 2

| Encapsulation efficiency of interferon alpha into liposomes | |
|---|---|
| **Formula No.** | **Encapsulation efficiency %±s.d. (n=5)** |
| 1 | 59.1±10.4 |
| 2 | 55.9±1.5 |

## B. Preparation of biphasic multilamellar lipid vesicles with a central lipophilic core compartment

1° Formation of the anhydrous plastic proliposome gel

The procedure used to form the anhydrous plastic proliposome gel is the same as the procedure described previously. As mentioned before, this gel contains all the lipid phase ingredients used to form the lipid bilayers of the multilamellar lipid vesicle. It can be prepared and stored in advance in large quantities.

2° Formation of the central lipophilic core compartment

The central lipophilic core compartment is made from a lipophilic substance-in-water emulsion in the form of a droplet comprising on its surface a liposome-formation promoting agent adapted to stabilize the liposome-forming component and to promote the formation of lipid vesicles around the droplet.

The term "lipophilic substance", when used herein, is intended to designate oils or solid/semisolid lipophilic consistency enhancers which can be encapsulated into liposomes. Examples of oils which have successfully been encapsulated into liposomes include: olive oil, macadamia nut oil, rice bran oil, pumpkin seed oil, apricot kernel oil, hazelnut oil, rose hip seed oil, borage oil, tea tree oil, rocou oil, camellia oil, sweet almond oil, safflower oil, St. John's wort oil, lipodermol, pistachio nut oil, chia oil, kiwi seed oil, lesquerella oil, tiare oil, grape-seed oil, peanut oil, babassu oil, avocado oil, squalene, evening primrose oil, blackcurrent oil, pentaerythritol tetracaprylate/caprate, pentaerythritol tetraisostearate, stearyl octanoate and canola oil, jojoba oil, peanut oil, rice bran oil, cottonseed oil, sunflower oil, corn oil, walnut oil, avocado oil, peru balsam, clove oil and eugenol. Plant extracts based on oil have also been successfully incorporated into liposomes such as from comfrey, German chamomile, cola nut, pot marigold, silver birch, cola nut, gingko biloga, witch hazel, willow, guarana, arnica, horse chestnut, eucalyptus, meadow sweet, rosemary, phyt'lod, coffee, mate, tea and ivy. With regard to solid/semisolid lipophilic consistency enhancer ingredients, they can be selected from waxes, fatty alcohols, fatty acid esters, glyceryl stearate, petrolatum or combinations thereof. Specific examples of preferred consistency enhancers include beeswax, glyceryl tribehenate, glyceryl stearate, stearyl heptanoate, stearyl palmitate, cetyl alcohol, stearyl alcohol, myristyl myristate, behenyl erucate and cetyl palmitate.

The term "liposome-formation promoting agent" when used herein is intended to designate compounds that can be used to stabilize the liposome-forming component and promote the formation of lipid vesicles around the droplets. The obvious choice is surfactants. The surfactant used to coat the oil droplet or the solid/semisolid lipophilic consistency enhancer ingredients is important for the successful encapsulation of a lipophilic core into multilamellar lipid vesicles. About 30 different types of surfactants were screened and one family of surfactant gave the most acceptable results. This type of surfactant is a primary cationic emulsifier. The most preferred surfactant is linoleamidopropyl PG-Dimonium chloride phosphate. It is considered to be a synthetic phospholipid complex. This surfactant is commercially available from Mona Industries Inc.,Patterson, New Jersey under the trade name PHOSPHOLIPID EFA, abbreviated herein PEFA. Two other members of this family, also from Mona Industries, are stearamidopropyl PG-dimonium chloride phosphate, trade name PHOSPHOLIPID SV, and cocamidopropyl PG-dimonium chloride phosphate, trade name PHOSPHOLIPID PTC, are also acceptable but PEFA is preferred. Nonionic or amphoteric surf actants can also be used, such as naturally derived emulsifiers: PEG-60 almond glycerides, avocado oil diethanolamine, ethoxylated jojoba oil (PEG-40 Jojoba acid and PEG-40 Jojoba alcohol); polyoxyethylene derivatives: polyoxyethylene (20) sorbitan monooleate, polyoxyethylene (20) sorbitan monostearate; lanolin derivatives: polychol 20 (laneth 20), polychol 40 (laneth 40); neutral phosphate esters: PPG-cetyl ether phosphate, DEA oleth-3 phosphate. It is also possible to use anionic surfactants such as acylglutamates: TEA-cocoyl glutamate, sodium lauroyl glutamate, sodium hydrogenated tallow glutamate and sodium cocoyl glutamate.

When preparing the lipophilic substance-in-water emulsion, the hydrophilic ingredients are all incorporated in the water phase of the emulsion. The surfactant is dissolved in water and any other ingredient of hydrophilic character is added. Once the water phase of the emulsion has been prepared, the oil and/or solid/semisolid lipophilic ingredients are added to the water in a homogenizer for a period of time ranging from 5 to 30 minutes to obtain relatively small droplet size. Preferred droplet size ranges from 0.1 μm to 1 μm. The lipid phase melt is then heated and lipophilic substance-in-water emulsion is added and vigorously mixed by either vortexing or propeller mixing depending on the product size.

The formulation procedure described above can be easily adopted for large scale manufacturing. The propeller mixing approach can be directly scaled up by geometrically increasing the size of the vessel and the diameter of the propeller mixer. However, as the vessel size increases, the preferred set up would be a combination mixer such as a high intensity mixer with propeller mixer and a scraped surface agitator. In a large scale operation, the lipophilic substance-in-water emulsion is pumped from a first tank into a second tank containing the anhydrous plastic proliposome gel at the required temperature and mixed. The types of compounds which can be encapsulated into biphasic MLV include, but are not restricted to, prostaglandins, local anesthetic agents, proteins and peptides, antiviral agents, vitamins, antiinflammatory agents, antifungal agents, corticosteroids, plant extracts and amino acids. Specific examples include $PGE_1$, tetracaine, lidocaine, etidocaine, interferon alpha, interferon gamma, adenine-β-D-arabinofuranoside (Ara-A), 5-methoxymethyl-2'-deoxyuridine (MMUdR), vitamin A, vitamin C, vitamin E, provitamin $B_5$, azulene, allantoin, alpha-bisabolol and L-carnitine.

3° Characteristics of the biphasic multilamellar lipid vesicles having a central lipophilic core

With this type of multilamellar lipid vesicles, the encapsulation and coencapsulation of active ingredients with different physical chemical properties, either hydrophilic or lipophilic, is possible. Figures 3 and 4 provide schematic figures of multilamellar lipid vesicle structures having a central lipophilic core compartment. As it can be seen in Figure 3, the multilamellar vesicle, generally designated by reference numeral 10, comprises a series of liposome bilayers 12, 14 and 16 spaced apart to define a series of peripheral aqueous solution compartments 13 and 15. The lipid bilayer defining the smallest circle 18 forms an interior space 19 that constitutes the central core compartment. When an oil droplet is used, the surfactant acts to coat the surface of the droplet and stabilize it for encapsulation by a lipid bilayer. Following this first encapsulation, the remaining layers, once encapsulated, will form peripheral aqueous solution compartments. Hence, it is important that the size of the droplets be very small so as to be incorporated into the central core of the multilamellar lipid vesicle.

Similarly, when using a solid/semisolid lipophilic consistency enhancer, a central lipophilic core compartment is formed as shown in Figure 4. In this figure, the multilamellar vesicle, generally designated by reference numeral 20, comprises a plurality of liposome bilayers 22, 24 and 26 between which peripheral aqueous solution compartments 21 and 23 are formed. The lipid bilayer 28 having the smallest circumference, forms the central lipophilic core compartment 30. Central lipophilic core compartment 30 contains solid or semisolid consistency enhancers that are entrapped within the multilamellar lipid vesicle in the same manner as described before, that is through the use of a suitable surfactant.

With the multilamellar lipid vesicle of the present invention, oil droplets containing solubilized lipophilic biologically active compounds or oily plant extracts can be delivered through liposome encapsulation. Furthermore, the possibility of multicompartment encapsulation provides drug release over extended periods of time. Also, encapsulation of lipophilic solid/semisolid consistency enhancers into the central lipophilic core compartment provides enhanced viscosity to the final liposome composition. In this case, the addition of viscosity-increasing agents in the final liposome preparation can be avoided.

Overall, the preparation of multilamellar lipid vesicles with a central lipophilic core component provides a physically stable, uniform liposome composition. The composition has a viscosity that is suitable for topical administration and can be easily manufactured on a large scale.

## DESCRIPTION OF PREFERRED EMBODIMENTS

### A. Encapsulation of prostaglandins and prostaglandin analogs into multilamellar lipid vesicles

Liposome encapsulated prostaglandins and prostaglandin analogs can provide benefit in a number of treatment areas. The following table summarizes the utility of liposome encapsulated prostaglandins. The advantage of liposomal delivery of these prostaglandins is the more efficient, localized and targeted delivery to the desired tissues i.e. the skin, mucus membranes and surrounding tissues. The liposome system can be designed to provide a slow release depot at the target site within the skin or mucus membrane or localize the drug in the eye through bioadhesion and slow release or promote transdermal flux of the encapsulated drug.

| Prostaglandin | Effect and use |
|---|---|
| PGE1 | Vasodilatation: peripheral vascular diseases, ischaemic leg ulcers, wound healing, various skin conditions such as psoriasis, atopic dermatitis |
| PGE2 | cervical ripening or labour induction by local administration into the cervical canal or intravaginally; vasodilatation: peripheral vascular diseases |
| PGA and J series | anticancer and antiviral agents |
| PGF and derivatives | ocular hypotensive: glaucoma |

### A1. Encapsulation of $PGE_1$ into multilamellar lipid vesicles for the preparation of liposome composition used in the treatment of erectile dysfunctions

In order to overcome the problems associated with the administration of $PGE_1$ by injection, a new dosage form was developed which can be applied topically. $PGE_1$ is incorporated into liposomes using the method of the present invention. This promotes its penetration through the skin of a penis and delivers a clinically useful concentration of drug into the corpus cavernosum. Liposome encapsulation enhances the penetration of the encapsulated $PGE_1$ through the penile skin and at the same time protects the drug within the skin from premature metabolism before reaching the target site. These properties make liposomes a suitable delivery system for $PGE_1$ in the treatment of impotence.

$PGE_1$ is a lipid-soluble drug. Therefore, it can be encapsulated in the lipid bilayers and/or the central lipophilic core of the liposomes. The encapsulation efficiency of $PGE_1$ into liposomes is manipulated by varying lipid composition, lipid/drug ratio, pH and the concentration of other excipients. Specific liposome compatible penetration enhancers/release agents are employed in designing an optimal liposomal $PGE_1$ preparation for denial or transdermal delivery.

### 1° Encapsulation of $PGE_1$ in multilamellar lipid vesicles with a central aqueous core compartment

Multilamellar liposomes were prepared as follows: the lipid ingredients and the hydrophilic solvent propylene glycol were weighed into a glass vial or beaker and melted at 65 - 75°C by intermittent heating on a hot water bath. The hydrophilic ingredients were prepared separately as an aqueous solution. The lipid phase melt and aqueous phase solution were stored at -20°C and at 4°C respectively, until required. $PGE_1$ was stored as an ethanolic solution at -20°C. Prior to liposome formulation, the ethanol solvent was evaporated under nitrogen; the lipid phase melt was added to the solid $PGE_1$ and mixed thoroughly by intermittent warming and vortex agitation. The lipid phase melt containing $PGE_1$ and the aqueous phase were then heated to 55°C and the aqueous phase was added to the lipid phase melt and vigorously mixed by vortexing or propeller mixing depending on the product size.

The composition and encapsulation efficiency of selected liposomal $PGE_1$ formulations developed for transdermal delivery (Formulas #1, #2, #5 and #6-0.1%) and for dermal delivery (Formula #3 and 4) are shown in Table 3. The liposomal formulations for the transdermal delivery of $PGE_1$ contain saturated and/or unsaturated phospholipids, especially phosphatidylcholine, lysophosphatidylcholine, phosphatidylserine, phosphatidylethanolamine, glycolipids. The liposomes also contain cholesterol, saturated long chain fatty acids (e.g. stearic acid) to improve physical stability and appearance of the product. The phospholipids are in combination with a penetration enhancing agent such as monolauroyllysine and/or methyl salicylate to achieve predominantly transdermal delivery potential.

The liposomal formulations for the dermal delivery of $PGE_1$ can contain saturated and/or unsaturated phospholipids, especially phosphatidylcholine, lysophosphatidylcholine, phosphatidylserine, phosphatidylethanolamine, glycolipids and ceramides. The liposomes may also contain cholesterol. To achieve predominantly dermal delivery cutaneous release agents such as unsaturated fatty acids (oleic acid) or short chain fatty acids (lauric acid) is added.

### TABLE 3

#### Liposomal $PGE_1$ formulas

**FORMULA # 1**

| | |
|---|---|
| Phospholipon 90H | 15% |
| Cholesterol | 2% |
| Ascorbyl Palmitate | 0.05% |
| Phospholipon 80 | 1% |
| Prostaglandin $E_1$ | 0.05% |
| Propylene Glycol | 7% |
| Aqueous Phase F | q.s. |
| Encapsulation Efficiency: 16% | |

**FORMULA #2**

| | |
|---|---|
| Phospholipon 90H | 15% |
| Cholesterol | 1.5% |
| Ascorbyl Palmitate | 0.05% |

| | |
|---|---|
| Propylene glycol | 7% |
| Monolauroyllysine | 1% |
| Glycerol | 5% |
| Stearic Acid | 0.5% |
| Centrolex P | 0.3% |
| Prostaglandin $E_1$ | 0.05% |
| Aqueous Phase 16 | q.s. |

Encapsulation Efficiency: 81%

**FORMULA # 3**

| | |
|---|---|
| Phospholipon 90H | 15% |
| Cholesterol | 1.5% |
| Ascorbyl Palmitate | 0.05% |
| Propylene glycol | 7% |
| Monolauroyllysine | 1% |
| Glycerol | 5% |
| Lauric Acid | 0.5% |
| Centrolex P | 0.3% |
| Prostaglandin $E_1$ | 0.05% |
| Aqueous Phase 16 | q.s. |

Encapsulation Efficiency: 76%

**FORMULA # 4**

| | |
|---|---|
| Phospholipon 90H | 15% |
| Cholesterol | 1.5% |
| Ascorbyl Palmitate | 0.05% |
| Propylene glycol | 7% |
| Monolauroyllysine | 1% |
| Glycerol | 5% |
| Oleic Acid | 1% |
| Centrolex P | 0.3% |
| Prostaglandin $E_1$ | 0.05% |
| Aqueous Phase 16 | q.s. |

Encapsulation Efficiency: 67%

**FORMULA # 5**

| | |
|---|---|
| Phospholipon 90H | 15% |
| Cholesterol | 1.5% |
| Ascorbyl Palmitate | 0.05% |
| Propylene glycol | 7% |
| Monolauroyllysine | 1% |
| Glycerol | 5% |
| Stearic Acid | 0.5% |
| Centrolex P | 0.3% |
| Methylsalicylate | 2% |
| Prostaglandin $E_1$ | 0.05% |
| Aqueous Phase 16 | q.s. |

Encapsulation Efficiency: 33%

**FORMULA # 6 - 0.1%**

| | |
|---|---|
| Phospholipon 90H | 15% |
| Cholesterol | 1.5% |
| Ascorbyl Palmitate | 0.05% |
| Propylene glycol | 7% |
| Monolauroyllysine | 1% |
| Glycerol | 5% |
| Stearic Acid | 0.5% |
| Centrolex P | 0.3% |
| Methylsalicylate | 2% |
| Prostaglandin $E_1$ | 0.1% |
| Aqueous Phase 16 | q.s. |

**FORMULA # 7**

| | |
|---|---|
| Phospholipon 90H | 15% |
| Cholesterol | 1.5% |
| Ascorbyl palmitate | 0.05% |
| Propylene glycol | 7.0% |
| Monolauroyllysine | 1.0% |
| Glycerol | 5.0% |
| Stearic acid | 0.5% |
| Centrolex P | 0.3% |

| Prostaglandin E$_1$ | 0.05% |
| Calcium thioglycolate | 1.0% |
| Aqueous phase 16 | q.s. to 100% |

**CONVENTIONAL (NON-LIPOSOMAL) FORMULA**

| Prostaglandin E$_1$ | 0.05% |
| Dermabase | q.s. |

| AQUEOUS PHASE F | | AQUEOUS PHASE 16 | |
|---|---|---|---|
| NaCl | 0.9% | NaCl | 0.9% |
| Ethanol | 10% | Methylparaben | 0.1% |
| dd, H$_2$O | q.s. | Propylparaben | 0.02% |
| pH | 5.5 | Triethanolamine | 0.5% |
| | | dd, H$_2$O | q.s. |
| | | pH | 8.3 |

2. In vitro testing of percutaneous absorption of PGE$_1$

The rate of diffusion of PGE$_1$ from the liposome preparations across full thickness adult foreskin from circumcision surgery was investigated using Teflon® Flow-Thru Diffusion Cells (Crown Glass Co. Inc. Somerville, N.J.) which have a surface area for diffusion of 0.32 cm$^2$. The diffusion cells are designed such that fluid may be continuously pumped through them in order to maintain sink conditions. A phosphate buffer (7.5 mM Na$_2$HPO$_4$, 141.2 mM NaCl) isotonic with body fluids and having a pH of 7.2 was used as the perfusion fluid.

The diffusion cells were mounted in a PosiBloc™ Diffusion Cell Heater (Crown Glass Co. Inc., Somerville, N.J.) maintained at 32°C by a circulating water bath. The flow rate was 3 mL per hour. Each experiment was conducted for a period of 24 hours continuously. Liposome preparation (20 mg) containing 10 µg PGE$_1$ and $^3$H-prostaglandin E$_1$ as tracer, was applied to each cell at the beginning of each experiment. This amount is estimated to be roughly equivalent to the dose per unit surface area of the preparation (1.5g) that was applied on the shaft of the penis in the clinical trials. At least three replicates of each experiment were performed. The quantity of PGE$_1$ in each fraction was determined by liquid scintillation counting.

Skin penetration parameters were calculated from the steady state part of the cumulative flux plots as follows: a) Permeability Coefficient, P=J/∆C  where J=steady state slope and ∆C=concentration of PGE$_1$ in the donor chamber

b) Diffusion Coefficient, D=h$^2$/6L  where h=mean thickness of foreskin specimen=1mm and L=lag time in seconds.

For the treatment of impotence there is a requirement to deliver 5-40 µg PGE$_1$ (the equivalent amount delivered during intracavernous injection) transdermally. This amount should be delivered within short period (ideally within 1h) to be practically useful. The data obtained shows that PGE$_1$ can be delivered transdermally by appropriate liposomal formulas.

Table 4 shows the percutaneously delivered PGE$_1$ in vitro through human foreskin. Formulas #1, 2, 5 and 6-0.1% illustrate the formulation approach to develop a transdermal product.

TABLE 4

| Percutaneous absorption of $PGE_1$ from liposomal formulations in 24 hours | | |
| --- | --- | --- |
| Formula # | $\mu g/cm^2 \pm S.D.$ | % of Total applied±S.D. |
| 1 | 0.96±0.87 | 2.14±1.95 (n=3) |
| 2 | 1.39±1.56 | 3.87±4.34 (n=8) |
| 5 | 3.51±2.80 | 9.93±7.93 (n=6) |
| 6 - 0.1% | 6.73±3.97 | 8.74±5.15 (n=6) |
| 3 | 0.06±0.04 | 0.17±0.12 (n=3) |
| 4 | 0.34±0.55 | 0.89±1.43 (n=3) |
| Non-liposomal | 0.38±0.61 | 1.03±1.64 (n=3) |

The initial drug concentration used in these formulas was 0.05%. Effective drug concentration in these formulas can vary between 0.01-5%. As illustrated with formula #5 and 6-0.1%, increasing drug concentration to 0.1% doubled the amount of transdermally delivered $PGE_1$.

The percutaneous absorption parameters are shown in Table 5. The flux values obtained for the products correlated well with their in vivo efficacy assessed by doppler ultrasonography. The higher flux values indicate higher potency in the patients. Formulas #3 and 4 show low transdermal delivery potential.

TABLE 5

| *In vitro* skin penetration parameters | | | | |
| --- | --- | --- | --- | --- |
| FORMULA # | LAG TIME [h] | STEADY STATE FLUX $(J)_2$ [$\mu g/cm^2/h$] | DIFFUSION COEFFICIENT (D) [$cm^2/s$ x $10^{-8}$] | PERMEABILITY COEFFICIENT (P) [cm/h x$10^4$] |
| 1 | 8.5h | 0.059 | 5.50 | 1.18 |
| 2 | 7.9 | 0.083 | 5.86 | 1.66 |
| 5 | 7.7 | 0.191 | 6.01 | 3.82 |
| 6-0.1% | 9.5 | 0.427 | 4.87 | 4.27 |
| 3 | 10.6 | 0.004 | 4.37 | 0.02 |
| 4 | 12.8 | 0.029 | 3.62 | 0.58 |
| Nonliposomal | 15.1 | 0.042 | 3.07 | 0.84 |

Table 6 shows the extent of delivery of $PGE_1$ with the various formulas. Formulas #3 and 4 are examples for formulas to achieve predominantly cutaneous delivery.

TABLE 6

| Cutaneous delivery of PGE$_1$ from liposomal formulations | | | | | | |
|---|---|---|---|---|---|---|
| Formula # | Whole skin | | Stratum corneum | | Viable epidermis+dermis | |
| | µg/cm$^2$ | %* | µg/cm$^2$ | %* | µg/cm$^2$ | %* |
| 10 (n=3) | 7.11±2.63 | 15.98±3.87 | 0.85±0.84 | 1.84±1.63 | 6.99±3.39 | 14.80±4.07 |
| 18A (n=8) | 4.15±2.79 | 11.97±7.99 | 0.23±0.15 | 0.74±0.48 | 3.83±2.72 | 11.16±8.57 |
| 29 (n=6) | 4.96±2.15 | 14.18±6.32 | 0.32±0.20 | 0.93±0.66 | 4.27±3.21 | 12.11±8.88 |
| 29-01 (n=6) | 20.25±7.44 | 26.16±8.30 | 0.54±0.23 | 0.71±0.28 | 15.54±8.56 | 20.46±8.21 |
| 21 (n=3) | 1.71±0.83 | 4.85±1.73 | 0.17±0.08 | 0.50±0.27 | 0.89±0.78 | 0.251±2.13 |
| 27 (n=3) | 5.82±4.21 | 14.89±10.18 | 0.14±0.06 | 0.37±0.15 | 4.70±4.43 | 11.80±10.33 |

\* % of total applied

The transdermal versus dermal delivery potential of these formulations is reflected in the T/D ratio (Table 7) calculated from the mean (µg) transdermally absorbed drug and the mean (µg) cutaneously (whole skin count) delivered PGE$_1$ amount.

TABLE 7

| Transdermal versus dermal delivery ratios for liposomal PGE$_1$ products | |
|---|---|
| Formula # | Transdermal/Dermal delivery ratios |
| 1 | 0.135 |
| 2 | 0.335 |
| 5 | 0.708 |
| 6-0.1% | 0.332 |
| 3 | 0.035 |
| 4 | 0.060 |

4. Physical stability of liposomal products

Physical stability of the liposomal formulations was assessed by visual and microscopic evaluation immediately after preparation and at weekly intervals following storage at room temperature, or at 4°C.

The liposomal formulas were physically stable even after prolonged storage at room temperature and at 4°C. The formulations were completely homogenous with no signs of phase separation macroscopically. Under the light microscope, numerous multilamellar liposomes of varying sizes and mostly spherical shape were seen. There was no increase in fusion or aggregation products (indicators of liposome instability) upon storage.

4. Stability of PGE$_1$ in liposomal formulations

Stability of PGE$_1$ was analyzed by thin-layer chromatographic (TLC) and radiometric methods.

4.1. TLC Assay

Degradation of PGE$_1$ was assessed by TLC analysis of the diffusion cell fractions and the liposomal PGE$_1$ formulation (after storage at 4°C for 2 weeks). The aqueous fractions from diffusion cell experiments were pooled and freeze-dried. The PGE$_1$ was extracted with methanol, filtered through a Millipore filter and concentrated prior to spot-

ting. The liposomal PGE$_1$ formulation was diluted with either methanol or dioxane prior to spotting. The mobile phase used was Benzene:Dioxane:Acetic acid (20:20: 1v/v) while color reagent was Absolute Ethanol:4-Methoxybenzaldehyde:Concentrated Sulphuric Acid (20:20:2v/v). After the chromatographic run the plate was allowed to dry, sprayed with the color reagent, allowed to dry again for at least 15 minutes and heated in the oven at 85°C for 10 minutes for full color development. The PGE$_1$ spots appeared as dark brown spots, identical to a co-chromatographed PGE$_1$ standard.

### 4.2. Radiometric assay

Semi-quantitative assay of the thin-layer chromatograms was done by liquid scintillation counting of the PGE$_1$ spots and the rest of the chromatogram and expressing PGE$_1$ concentration as a percentage of total counts obtained in the chromatogram.

PGE$_1$ was positively identified on thin-layer chromatograms of the liposomal PGE$_1$ formulation after storage for 2 weeks in the refrigerator. Based on visual examination of the intensity of color development and band width, little or no PGE$_1$ degradation appeared to have occurred.

### 5. Encapsulation efficiency and leakage studies

Liposome encapsulation of PGE$_1$ is proposed to enhance its stability hence the greater the encapsulation efficiency, the more stable the drug will be in the formulation.

The encapsulation efficiency of PGE$_1$ into liposomes was determined by liquid scintillation counting. Aliquots of the liposomal cream were diluted with the aqueous phase and centrifuged at 200,000 xg for 60 minutes. The supernatant was separated from the pellet (liposomes) and the weights recorded. Calculated amounts of the supernatant and pellet were dissolved in scintillation cocktail and counted. The concentration of PGE$_1$ in each fraction were derived from the total activity (dpm) in each fraction.

$$\text{Encapsulation Efficiency} = \text{Concentration of PGE}_1 \text{ in pellet/(Concentration in pellet and supernatant)}$$

Encapsulation of efficiency studies were repeated at weekly intervals for 2 weeks to determine leakage of encapsulated drug from liposomal formulations stored either in the refrigerator or at room temperature.

### 6. Clinical evaluations

The protocols for the human studies were accepted by the Ethics Committee on Human Experimentation at the University of Saskatchewan.

Patients included in these studies were diagnosed as having impotence of primarily organic origin. This group of patients is the most difficult to treat and they usually require higher doses of PGE$_1$. The rationale for using this "difficult" group is to decrease the possibility to obtain placebo responses.

### 6.1. Double blind crossover trial

Six patients diagnosed as having impotence of predominantly organic etiology and shown to respond to intracavernous PGE$_1$ injection were enrolled in the study after proper consent.

A double blind, randomized, crossover design was employed to test a liposomal PGE$_1$ against a liposomal placebo. 1.5g of each cream was applied to the shaft of the penis at weekly intervals. Prior to this, a small amount was applied to a non-hairy part of the arm to test for adverse reactions. If none occurred within 60 minutes, the patient then proceeded with the test later in the day. The cream was washed off after 1 hour and prior to intercourse, if an erection was obtained. The patient was requested to note the onset, duration and quality of response as well as any side effect on the response sheets provided.

The study indicated that 5 out of 6 patients responded to the test preparation to various degrees (Response ranking ≥ 1, Table 8). Three patients responded with high scores (response ranking ≥ 2). Response to the placebo was practically zero.

## TABLE 8

### Pilot clinical study using Formula # 6 (0.1%)

### in patients with organic impotence

| Patient # | Response ranking | |
|---|---|---|
| | Liposomal PGE$_1$ | Placebo |
| 1 | 3 | 0 |
| 2 | 3 | 0 |
| 3 | 2 | 0 |
| 4 | 1 | 0 |
| 5 | 1 | 1 |
| 6 | 0 | 0 |

| | Response ranking |
|---|---|
| 0 | No effect whatsoever observed. |
| 1 | Slight effect i.e. slight enlargement of the penis. No erection. |
| 2 | Erection obtained but not sufficient for intercourse. |
| 3 | Good erection obtained. Intercourse attempted but not satisfactory. |
| 4 | Good erection obtained. Satisfactory intercourse. |

6.2 Doppler ultrasonography study

Doppler ultrasonography was used to assess the degree of arterial dilatation and measure penile arterial blood flow following application of transdermal liposomal PGE$_1$. Three liposomal PGE$_1$ preparations and a placebo control were tested. Test preparations were administered at no less than 2 week intervals. After initial sonographic examination to determine blood flow in the flaccid penis, the test preparation was applied on the shaft and glans penis after which an occlusive wrapping was placed around the penis. The penile blood flow was monitored at 15 minute intervals for 1 hour.

The doppler ultrasonography study on patients with predominantly organic impotence showed increased penile blood flow after topical liposomal PGE$_1$ application (Table 9), Liposome formula #5 showed the greatest efficacy, with a peak flow of 32 cm/s in Patient #4. The control liposome #5 formulation (without PEG$_1$) also exhibited an increase in penile blood flow. The #5 liposome preparation is equivalent to the #2 plus a penetration enhancing agent, methyl salicylate. It is likely that this penetration enhancer increases penile blood flow to a small degree by itself and is synergistic with liposomal PGE$_1$. The in vitro percutaneous absorption data correlated very well with the doppler results. In vitro #5 liposomal PGE$_1$ gave the highest percutaneous flux followed by #2 and lastly, the #7 liposomal PGE$_1$ formulation.

TABLE 9

| Peak systemic flow velocity (cm/s): deep cavernosal artery | | | | | | |
|---|---|---|---|---|---|---|
| Patient #/ Test # | Formulation # | Baseline | 0-15 min | 16-30 min | 31-45 min | 46-60 min |
| 1/1 | 18A-PGE$_1$ | 0 | 0 | 5 | 10 | 8 |
| 1/2 | 18A-Placebo | 0 | 0 | 0 | 0 | 0 |
| 2/1 | 18A-PGE$_1$ | 0 | 0 | 0 | 15 | 14 |
| 2/2 | 29-PGE$_1$ | 0 | 10 | 17 | 20 | 22 |
| 3/1 | 29-PGE$_1$ | 0 | 0 | 8 | 13 | 9 |
| 3/2 | 28-PGE$_1$ | 0 | 0 | 0 | 0 | 0 |
| 4/1 | 29-PGE$_1$ | 10 | 18 | 17 | 32 | 16 |
| 4/2 | 29-MSC | 0 | 12 | 10 | 10 | 18 |
| 5/1 | 28-PGE$_1$ | 0 | 0 | 0 | 0 | 0 |
| 5/2 | 29-PGE$_1$ | 0 | 0 | 0 | 6 | 6 |
| 0 No ultrasonographically detectable flow. MSC Methylsalicylate | | | | | | |

Other possible uses for topically applied liposomal PGE$_1$ include the preparation of transdermal formulations for the treatment of impotence as well as the preparation of dermal formulations for the treatment of various skin conditions such as atopic skin, psoriasis, peripheral vascular diseases, wound healing and ischaemic lesions.

## B. TROPICAL LIPOSOME-ENCAPSULATED INTERFERON ALPHA FOR THE TREATMENT OF GENITAL PAPILLOMAVIRUS INFECTIONS

Methods

**Liposome preparation**

The method used in A. was also used to incorporate IFN alpha which is included in the aqueous phase which is used to hydrate the proliposome gel by mixing at a temperature not exceeding 40°C.

**In situ hybridization**

For the determination of the presence of HPV DNA (types 6 and 11, types 16 and 18, and types 31, 33, and 35) in paraffin-embedded tissue sections from biopsies of patients, ViraType™ in situ Human Papillomavirus Tissue Hybridization Kit (Life Technologies, Inc., Gaithersburg, MD) was used.

**Immunostaining**

Biopsy sections before and after treatment with liposomal IFN alpha were immunostained for the presence of absorbed IFN by using mouse anti-human IFN monoclonal antibody (Boehringer Mannheim Biochemica) and alkaline phosphatase labelled secondary antibody in the form of AS/AP™ universal Mouse Immunostaining Kit (BIO/CAN Scientific Inc., Mississauga, Ont.).

**Clinical study**

The first patient was a 30-year old female with a three-year history of recurrent genital HPV with cervical involvement (CIN I). On examination prior to instituting topical IFN alpha (F#2) therapy, large confluent plaques and separate verrucous and filiform papules typical for condylomata acuminata were present on the labia majora, labia minora and upper thighs. Histopathology showed condyloma with mild intraepithelial atypia. VIRA-PAP was positive for HPV 6/11. In situ hybridization showed positivity for HPV 6/11, faint reactivity for HPV 31, 33 and 35, negative for HPV 16/18.

Treatment protocol was as follows 3x10[6] IU/week liposome encapsulated Intron A for 12 weeks, then 10x10[6] IU/week for 6 weeks; 1g twice a day externally, and 1g once a day intravaginally. The second patient was a 23-year old male with a two-year history of genital HPV. On examination there were multiple flat flesh-colored papules scattered over the shaft, foreskin and glans of the penis. Almost all of the lesions were acetowhite after five minutes of acetic acid soaking. Histopathology showed acanthosis, koilocytes, inflammation. VIRA-PAP was positive for HPV 6/11, negative for HPV 16/18, 31,33 and 35. In situ hybridization was negative for 6/11; 16/18; 31, 33 and 35. Treatment protocol 3x10[6] IU/week liposome encapsulated Intron A for 8 weeks, 1g twice a day externally. Assessment in both cases was carried out by monitoring the size and number of lesions, histopathology and in situ hybridization.

Results

## Cutaneous penetration of $^{125}$I-IFN

The absorption of IFN from various liposomal formulations into human breast skin in vitro was investigated using iodinated IFN as a label. The range of concentration of IFN investigated was 20-80 MU/g preparation. The usual applied dose was 0.1 g preparation/0.32 $cm^2$ skin surface area in the diffusion cell, which corresponds to about 6-28 MU IFN per $cm^2$ skin surface area depending on the concentration tested. The experiments utilized human breast skin from mammoplasty.

The cutaneous penetration of IFN from various liposomal formulas containing 20 MU/g preparation are shown in Tables 10A and 10B.

## TABLE 10A

### Cutaneous absorption of $^{125}$I-IFN from liposomal formulations at 48 h steady state. The total amount of IFN in whole skin (all skin layers together) is presented

Drug concentration in product:    $20x10^6$ IU IFN/g product
Dose/unit skin surface area:    $6x10^6$ IU IFN/$cm^2$
Skin used:    0716-92
Tracer:    $^{125}$I-IFN
Duration of experiment:    48 h
The values represent the mean $\pm$ S.D. (n=4)

| FORMULAS | IFN in WHOLE skin | | |
|---|---|---|---|
| | ng/$cm^2$ | IU/$cm^2$ | % of total applied |
| F#2 | 491.75$\pm$136.39 | 110,380$\pm$30,615 | 2.00$\pm$0.55 |
| F#3 | 1004.84$\pm$269.18 | 225,553$\pm$60,424 | 3.47$\pm$0.93 |
| F#5 | 783.67$\pm$52.49 | 175,904$\pm$11,783 | 2.70$\pm$0.18 |
| F#9 | 971.29$\pm$157.52 | 218,022$\pm$35,358 | 3.32$\pm$0.54 |
| F#10 | 822.58$\pm$202.53 | 183,022$\pm$45,120 | 3.56$\pm$0.88 |
| F#11 | 768.11$\pm$151.44 | 172,409$\pm$33,992 | 3.16$\pm$0.62 |
| Solution | 344.41$\pm$105.52 | 77,306$\pm$23,684 | 1.23$\pm$0.38 |

EP 0 711 148 B1

## TABLE 10B

Cutaneous absorption of $^{125}$I-IFN from liposomal formulations at 48 h steady state. The amount of IFN in the stratum corneum and in the deeper layers of skin (viable epidermis and dermis) is presented.

Drug concentration in product:     $20 \times 10^6$ IU IFN/g product

Dose/unit skin surface area:     $6 \times 10^6$ IU IFN/cm$^2$

Skin used:     0716-92

Tracer:     $^{125}$I-IFN

Duration of experiment:     48 h

The values represent the mean ±S.D. (n=4)

| FORMULAS | IFN in stratum corneum | | | IFN in viable epidermis+dermis | | |
|---|---|---|---|---|---|---|
| | ng/cm$^2$ | IU/cm$^2$ | % of total | ng/cm$^2$ | IU/cm$^2$ | % of total |
| F#2 | 239.63±136.39 | 53,787±28,833 | 0.95±0.51 | 213.90±33.04 | 48,013±74.15 | 0.87±0.13 |
| F#3 | 331.15±111.04 | 74,346±24,929 | 1.14±0.38 | 690.86±326.07 | 155,076±73,191 | 2.38±1.12 |
| F#5 | 375.57±183.82 | 84,308±41,263 | 1.29±0.63 | 426.95±211.60 | 95,833±47,495 | 1.47±0.73 |
| F#9 | 554.07±188.27 | 124,343±42,250 | 1.94±0.66 | 528.65±202.55 | 118,665±45,467 | 1.85±0.71 |
| F#10 | 426.17±192.54 | 95,750±43,260 | 1.85±0.83 | 418.26±78.44 | 93,180±17.475 | 1.81±0.34 |
| F#11 | 485.26±228.59 | 108,955±51,324 | 1.99±0.94 | 322.52±72.93 | 72,393±16,369 | 1.32±0.30 |
| Solution | 155.06±65.61 | 34,815±14,730 | 0.56±0.23 | 178.14±23.40 | 39,985±5,257 | 0.64±0.08 |

With the dosage regimen used in this study the applied dose was around 6 MU/cm$^2$. The absorption of IFN from the various liposomal formulas was fairly similar, 2.00-3.56% of the total applied when the whole (unstripped skin was analyzed (Table 10A). This corresponds to about 110,000-218,000 IU IFN/cm$^2$ skin. When the stripped skin was analyzed (Table 10B), the difference in the rate of absorption into the deeper layers, i.e. the viable epidermis and dermis, was noticeable. The amount of IFN delivered by Formulas #3, 5, 9, 10 and 11 was in the range of 72,000-155,000 IU/cm$^2$. The control IFN solution at the same concentration and dose delivered approximately 77,000 IU IFN/cm$^2$ into 'whole' skin (1.23±0.38% of total applied) and 40,000 IU/cm$^2$ into the deeper layers of skin (0.64%±0.08 of total). Similar results were obtained when more concentrated solutions were used.

Upon increasing the dose the amount of IFN absorbed also increased. Selected formulas with 40 MU/g were prepared and tested. In this case the applied dose was around 12 MU/cm$^2$. F# 5-40, F#10-40 and F#11-40 had the same lipid composition as before except the initial drug concentration was increased. The amount of absorbed IFN into skin reached about 0.5 MU/cm$^2$.

Table 11 indicates that among these three preparations F#10-40 delivered the highest amount of IFN, close to 600, 000 IU/cm$^2$. After stripping the skin the level of IFN in the skin treated with Formula #10-40 was still close to 0.5 MU/cm$^2$ F#5-40 and F#11-40 delivered somewhat lower quantities of IFN into the deeper layers of the skin.

## TABLE 11

Cutaneous absorption of $^{125}$IN-IFN from liposomal formulas containing 40MU/g IFN at 48 h steady state. The amount of IFN in whole skin (all skin layers together) and in the stratum corneum and in the deeper layers of skin are presented.

Drug concentration in product: 40x10$^6$ IU IFN/g product

Dose/unit skin surface area: 12x10$^6$ IU IFN/cm$^2$

Skin used: 0716-92

Tracer: $^{125}$I-IFN

Duration of experiment: 48 h

The values represent the mean ±S.D. (n=4)

**IFN in WHOLE skin**

| FORMULAS | ng/cm$^2$ | IU/cm$^2$ | % of total applied |
|---|---|---|---|
| F#5-40 | 1560.63±157.68 | 350,309±35,395 | 3.37±0.34 |
| F#10-40 | 2627.28±705.42 | 589,284±158,221 | 4.69±1.26 |
| F#11-40 | 1830.23±625.73 | 409,837±140,118 | 3.40±1.16 |

| FORMULAS | IFN in stratum corneum | | | IFN in viable epidermis-dermis | | |
|---|---|---|---|---|---|---|
| | ng/cm$^2$ | IU/cm$^2$ | % of total | ng/cm$^2$ | IU/cm$^2$ | % of total |
| F#5-40 | 808.10±296.25 | 181,459±66,523 | 1.74±0.64 | 734.12±174.67 | 164,785±39,207 | 1.58±0.37 |
| F#10-40 | 460.02±282.00 | 103,176±63,250 | 0.82±0.50 | 2141.61±959.46 | 480,350±215,202 | 3.82±1.71 |
| F#11-40 | 952.06±352.05 | 213,244±78,851 | 1.77±0.65 | 930.59±676.30 | 208,385±151,441 | 1.72±1.25 |

With F#5 and F#10 the concentration of IFN was further increased to 80 MU/g to determine whether more drug can be delivered. Table 12 shows that F#10-80 delivered close 0.9 MU/cm$^2$ into the skin. The viable epidermis and dermis contained about 0.7 MU/cm$^2$. It is noteworthy that F#10 in general delivered a greater proportion of IFN into the deeper layers of the skin. This effect is more pronounced at 40 MU/g and at 80 MU/g product concentration (Tables 11 and 12).

Estimation of antiviral activity in skin treated with liposomal IFN alpha.

## TABLE 12

Cutaneous absorption of $^{125}$I-IFN from liposomal formulas containing 80MU/g IFN at 48 h steady state. The amount of IFN in whole skin (all skin layers together) and in the stratum corneum and in the deeper layers of skin are presented.

Drug concentration in product:      $80 \times 10^6$ IU IFN/g product
Dose/unit skin surface area:      $28 \times 10^6$ IU IFN/cm$^2$
Skin used:      0716-92
Tracer:      $^{125}$I-IFN
Duration of experiment:      48 h
The values represent the mean $\pm$S.D. (n=4)

| FORMULAS | IFN in WHOLE skin | | |
|---|---|---|---|
| | ng/cm$^2$ | IU/cm$^2$ | % of total applied |
| F#5-80 | 3452.49±929.00 | 773,106±208,028 | 2.75±0.74 |
| F#10-80 | 3867.71±1375.89 | 866,084±308,099 | 3.21±1.41 |

| FORMULAS | IFN in stratum corneum | | | IFN in viable epidermis-dermis | | |
|---|---|---|---|---|---|---|
| | ng/cm$^2$ | IU/cm$^2$ | % of total | ng/cm$^2$ | IU/cm$^2$ | % of total |
| F#5-80 | 1976.43±1575.20 | 442,587±352,728 | 1.57±1.25 | 1216.18±370.58 | 272,335±82,983 | 0.97±0.29 |
| F#10-80 | 728.19±610.93 | 163,058±136,800 | 0.60±0.50 | 3080.23±1196.41 | 689,746±267,210 | 2.55±0.99 |

Measuring cutaneous penetration by radioactivity is an efficient way of comparing several different formulations and calculate approximate levels of protein in the skin. However, this method cannot answer the question whether the protein absorbed is intact and active.

In order to get a better understanding whether the radioactive label represents active protein, antiviral assays with the skin samples treated with liposomal IFN (20 MU/g) were carried out. These antiviral assays indicated that there was antiviral activity in the skin with each liposomal formula tested (Table 13). The values obtained by antiviral assay were about ten times lower than the amount of IFN detectable in skin by the radioactive method for all formulas except F#10. In case of F#10 the antiviral activity corresponded well with the result from the radioactive experiments (compare Table 10A and Table 13).

TABLE 13

| Estimation of antiviral activity in skin treated with liposomal IFN alpha in vitro | |
| --- | --- |
| Formula Number | Total IFN in breast skin at 48 h (IU/cm$^2$) |
| F#2 | 17,200 |
| F#3 | 26,600 |
| F#5 | 21,900 |
| F#10 | 219,000 |
| F#9 | 17,200 |
| F#11 | 17,200 |
| IFN solution | 12,000 |

**Delivery of IFN alpha into surgically removed genital wart tissue**

Excised condyloma tissues were treated with low-dose liposomal IFN (F#10-80) to obtain preliminary information about the rate of drug delivery. The difficulty of in vitro experiments with wart tissues is the localization of adequate quantities of product on the surface of the lesions. The two dose regimens used (0.5 MU/lesion and 2 MU/lesion, approximately) was required to form a sufficiently thick layer of the product on the surface of the tissue and any additional amount would not be accommodated (it would fall off). The results of these experiments are shown in Table 14.

Seven excised condyloma lesions (average diameter 5 mm) were treated with an average of 0.5 MU dose of F#10-80. The amount of IFN delivered into the lesions was 34,400±18,100 IU (6.5% of the total amount applied). After determining the radioactivity in the 'whole' lesions (i.e. after thorough washing and blotting of the warts), the outer layers were removed by tape stripping and the radioactivity remaining in the deeper layers was determined. The average amount of IFN in the deeper layer was 29,000±15,300 IU (5.5% of the total amount applied).

Three condyloma lesions were used for the experiments with the higher dose (2 MU/lesion) treatment. The total amount of IFN delivered was 323,500±235,100 IU (14.8% of total) and the remaining radioactivity after stripping of the warts was 286,500±208,900 IU (9.5% of total).

These experiments show potential for this formulation to deliver sufficient quantities of drug. However, by improving the experimental set up a better estimation of the delivery rate into wart tissue could be obtained. Some of the difficulties in these experiments are the availability of tissues of similar sizes, the application of the liposome cream to the lesions, the incubation set-up and the analysis of the tissues.

Treatment of patients with liposome encapsulated IFN alpha (F#2)

After several weeks of therapy of the female patient there was softening and thinning of the condyloma and also lightening of the hyperpigmentation. Three lesions removed under local anesthesia from the right upper thigh at tenth week of therapy were still resolved at the end of a 12-week course of liposomal interferon treatment and the area continued to show no sign of recurrence seven weeks after removal. Colposcopy one month following discontinuation of liposomal interferon therapy showed no evidence of HPV involvement of the cervix. In case of the male patient, several lesions on the distal foreskin and glans resolved after two weeks of liposomal interferon twice a day. Multiple lesions remained after eight weeks of therapy, when the patient moved from the area and was lost to follow-up. The lesions which resolved within the first two weeks remained gone during the observation period.

The pretreatment biopsy sections were not greatly different from the post-treatment sections, both showing acanthosis and papillomatosis, however koilocytic changes were less apparent in the post-treatment sections. In situ hybridization of tissue sections obtained by biopsy 18 week post-treatment of the female patient showed only a relatively weak, focal staining for HPV 6 and 11 in rare superficial cells as opposed to pretreatment, when the test showed numerous foci of positively staining nuclei within superficial layers of the epithelium. These latter results might indicate the elimination of HPV virus from the wart tissues.

We conclude that liposomes are a suitable delivery system for IFN alpha and a dermatologically acceptable dosage form. Our preliminary clinical studies indicate the efficacy of topically applied liposome encapsulated IFN alpha in the treatment of genital HPV infections.

**B. Encapsulation of lipophilic solid or liquid active ingredient or consistency enhancer into the central core compartment of multilamellar lipid vesicles**

1. Formulation procedure

Multilamellar liposomes were prepared as follows: the lipid ingredients and the hydrophilic solvent propylene glycol are weighed into a glass vial or beaker and melted at 65 - 75°C by intermittent heating on a hot water bath.

The hydrophilic ingredients and the oil droplets or solid/semisolid lipophilic ingredients are prepared separately as an oil in water (o/w) emulsion. This intermediate product is called System A. System A contains a suitable surfactant, the key ingredient to coat the surface of the lipophilic droplets and stabilize them for encapsulation into liposomes. System A is prepared by dissolving the surfactant in distilled water and adding the oil and/or solid/semisolid lipophilic ingredients to the water at 60-80°C in a homogenizer at 20-80 psig for 5-30 minutes to obtain small droplet size (<about 0.5 $\mu$m).

The lipid phase melt (which may or may not contain $PGE_1$) is heated to 55°C and System A is added to the lipid phase melt and vigorously mixed by vortexing or propeller mixing depending on the product size.

The following formulas have been prepared using this procedure.

| 1. Topical liposomal prostaglandin $E_1$ | |
|---|---|
| | % (w/w) |
| Phase A: Lipid phase: | |
| Phospholipon 90H | 15.0 |
| Cholesterol | 1.5 |
| Ascorbyl Palmitate | 0.05 |
| Monolauroyllysine | 1.0 |
| Stearic Acid | 0.5 |
| Centrolex P | 0.3 |
| Methyl salicylate | 2.0 |
| Prostaglandin $E_1$ | 0.05 |
| Propylene glycol | 7.0 |
| Phase B: Aqueous Phase | q.s. to 100 |
| Sodium chloride | 0.9 |
| Triethanolamine | 0.5 |
| Glycerol | 5.0 |
| Methylparaben | 0.1 |
| Propylparaben | 0.02 |
| Distilled water | 98.48 |

| 2. Topical liposomal product with encapsulated oil droplet | |
|---|---|
| | % (w/w) |
| Phase A: Lipid Phase: | |
| Phospholipon 90H | 5.0 |
| Cholesterol | 2.0 |
| Stearic acid | 1.0 |
| System A: | |
| Part 1: Distilled water | q.s. to 100 |
| PEFA | 4.0 |
| Methylparaben | 0.15 |
| Propylparaben | 0.05 |
| Part 2: Olive oil | 10.0 |

| 3. Topical liposomal product with encapsulated consistency enhancer | |
|---|---|
| | % (w/w) |
| Phase A: Lipid phase: | |
| Phospholipon 90H | 5.0 |
| Cholesterol | 2.0 |
| Stearic acid | 1.0 |
| System A: | |
| Part 1: Distilled water | q.s. to 100 |
| PEFA | 4.0 |
| Methylparaben | 0.15 |
| Propylparaben | 0.05 |
| Part 2: Glyceryl stearate | 1.0 |
| Cetyl alcohol | 0.6 |
| Synthetic beeswax | 0.28 |

| 4. Topical liposomal product with encapsulated oil droplet and consistency enhancer | |
| --- | --- |
| | % (w/w) |
| Phase A: Lipid phase: | |
| Phospholipon 90H | 5.0 |
| Cholesterol | 2.0 |
| Stearic acid | 1.0 |
| System A: | |
| Part 1: Distilled water | q.s. to 100 |
| PEFA | 4.0 |
| Methylparaben | 0.15 |
| Propylparaben | 0.05 |
| Part 2: Olive oil | 10.0 |
| Glyceryl stearate | 1.0 |
| Cetyl alcohol | 0.6 |
| Synthetic beeswax | 0.28 |

| 5. Topical liposomal product with encapsulated oily plant extract and consistency enhancer | |
| --- | --- |
| | % (w/w) |
| Phase A: Lipid phase: | |
| Phospholipon 90H | 5.0 |
| Cholesterol | 2.0 |
| Stearic acid | 1.0 |
| System A: | |
| Part 1: Distilled water | q.s. to 100 |
| PEFA | 4.0 |
| Methylparaben | 0.15 |
| Propylparaben | 0.05 |
| Part 2: German chamomile oily extract | 5.0 |
| Glyceryl stearate | 1.0 |
| Cetyl alcohol | 0.6 |
| Synthetic beeswax | 0.28 |

| 6. Topical liposomal product with encapsulated oil-soluble cosmetic active ingredient and consistency enhancer | |
|---|---|
| | % (w/w) |
| Phase A: Lipid phase: | |
| Phospholipon 90H | 5.0 |
| Cholesterol | 2.0 |
| Stearic acid | 1.0 |
| System A: | |
| Part 1: Distilled water | q.s. to 100 |
| PEFA | 4.0 |
| Methylparaben | 0.15 |
| Propylparaben | 0.05 |
| Part 2: Vitamin E | 10.0 |
| Glyceryl stearate | 1.0 |
| Cetyl alcohol | 0.6 |
| Synthetic beeswax | 0.28 |

| 7. Topical liposomal product with encapsulated lipophilic drug in multicompartments and with encapsulated oil droplet and consistency enhancer | |
|---|---|
| | % (w/w) |
| Phase A: Lipid phase: | |
| Phospholipon 90H | 5.0 |
| Cholesterol | 2.0 |
| Stearic acid | 1.0 |
| Prostaglandin $E_1$ | 0.05 |
| System A: | |
| Part 1: Distilled water | q.s. to 100 |
| PEFA | 4.0 |
| Methylparaben | 0.15 |
| Propylparaben | 0.05 |
| Part 2: Olive oil | 10.0 |
| Glyceryl stearate | 1.0 |
| Cetyl alcohol | 0.6 |
| Synthetic beeswax | 0.28 |
| Prostaglandin $E_1$ | 0.05 |

| 8. Topical liposomal product with encapsulated lipophilic drug in multicompartments and with encapsulated oil droplet and consistency enhancer | |
|---|---|
| | % (w/w) |
| Phase A: Lipid phase: | |
| Phospholipon 90H | 5.0 |
| Cholesterol | 2.0 |
| Stearic acid | 1.0 |
| Tetracaine | 1.0 |
| System A: | |
| Part 1: Distilled water | q.s. to 100 |
| PEFA | 4.0 |
| Methylparaben | 0.15 |
| Propylparaben | 0.05 |
| Part 2: Olive oil | 10.0 |
| Glyceryl stearate | 1.0 |
| Cetyl alcohol | 0.6 |
| Synthetic beeswax | 0.28 |
| Tetracaine | 1.0 |

| 9. Topical liposomal product with encapsulated protein drug and with encapsulated oil droplet and consistency enhancer | |
| --- | --- |
| | % (w/w) |
| Phase A: Lipid phase: | |
| Phospholipon 90H | 5.0 |
| Cholesterol | 2.0 |
| Monolauroyllysine | 2.0 |
| System A: | |
| Part 1: Phosphate buffer | q.s. to 100 |
| PEFA | 4.0 |
| Methylparaben | 0.15 |
| Propylparaben | 0.05 |
| Interferon alpha | 20 MU |
| Part 2: Olive oil | 10.0 |
| Glyceryl stearate | 1.0 |
| Cetyl alcohol | 0.6 |
| Synthetic beeswax | 0.28 |

| 10. Topical liposomal product with encapsulated antiviral drug combination with or without consistency enhancer | |
| --- | --- |
| Phase A: Lipid phase: | |
| Phospholipon 90H | 10.0 |
| Phospholipon 90 | 0.5 |
| Cholesterol | 1.0 |
| Stearic acid | 1.0 |
| Propylene glycol | 7.0 |
| Ara-A | 1.0 |
| MMUdR | 1.0 |
| Phase B: Aqueous phase F | q.s. to 100 |
| **OR:** | |
| Phase B: System A: | q.s. to 100 |
| Part 1: Distilled water | q.s. to 100 |
| PEFA | 4.0 |
| Methylparaben | 0.15 |

(continued)

| 10. Topical liposomal product with encapsulated antiviral drug combination with or without consistency enhancer | |
| --- | --- |
| Propylparaben | 0.05 |
| Part 2: Canola oil | 10.0 |

| 11. Topical liposomal product with encapsulated oil-soluble cosmetic active ingredients (vitamins) and consistency enhancer | |
| --- | --- |
| Phase A: Lipid phase: | |
| Phospholipon 90H | 5.0 |
| Cholesterol | 2.0 |
| Stearic acid | 1.0 |
| Ascorbyl palmitate | 0.5 |
| System A: | |
| Part 1: Distilled water | q.s. to 100 |
| PEFA | 4.0 |
| Methylparaben | 0.15 |
| Propylparaben | 0.05 |
| Part 2: Vitamin E | 10.0 |
| Vitamin A | 2.0 |
| Glyceryl stearate | 1.0 |
| Cetyl alcohol | 0.6 |
| Synthetic beeswax | 0.28 |

| 12. Topical liposomal product with encapsulated oil and oil-soluble cosmetic active ingredient and consistency enhancer | |
|---|---|
| Phase A: Lipid phase: | |
| Phospholipon 90H | 5.0 |
| Cholesterol | 2.0 |
| System A: | |
| Part 1: Distilled water | q.s. to 100 |
| PEFA | 4.0 |
| Methylparaben | 0.15 |
| Propylparaben | 0.05 |
| Part 2: Azulenol | 0.1 |
| Rose hip seed oil | 5.0 |
| Glyceryl stearate | 1.0 |
| Cetyl alcohol | 0.6 |
| Synthetic beeswax | 0.28 |

| 13. Topical liposomal product with encapsulated oil and oil-soluble cosmetic active ingredient and consistency enhancer | |
|---|---|
| Phase A: Lipid phase: | |
| Phospholipon 90H | 5.0 |
| Cholesterol | 2.0 |
| System A: | |
| Part 1: Distilled water | q.s. to 100 |
| PEFA | 4.0 |
| Methylparaben | 0.15 |
| Propylparaben | 0.05 |
| Part 2: Alpha bisabolol | 10.0 |
| Rocou ami oil | 3.0 |
| Glyceryl stearate | 1.0 |
| Cetyl alcohol | 0.6 |
| Synthetic beeswax | 0.28 |

| 14. Topical liposomal product with encapsulated protein drug and consistency enhancer | |
|---|---|
| | % (w/w) |
| Phase A: Lipid phase: | |
| Phospholipon 90H | 5.0 |
| Cholesterol | 2.0 |
| Monolauroyllysine | 2.0 |
| System A: | |
| Part 1: Phosphate buffer | q.s. to 100 |
| PEFA | 4.0 |
| Methylparaben | 0.15 |
| Propylparaben | 0.05 |
| Interferon gamma | 2 MU |
| Part 2: Glyceryl stearate | 1.0 |
| Cetyl alcohol | 0.6 |

When using compounds with acidic hydrophilic groups (phosphato, sulfato, etc.) the obtained SPLVs will be anionic; with basic groups such as amino, cationic liposomes will be obtained; and with polyethylenoxy or glycol groups neutral liposomes will be obtained. The size of SPLVs varies widely. The range extends from about 100nm to about 10,000 nm (10 microns) and usually about 100 nm to about 1500 nm.

Virtually any bioactive compound can be entrapped within a SPLV (entrapped is defined as entrapment within the aqueous compartment or within the membrane bilayer).

TABLE 14

Treatment of excised condyloma tissues with low-dose topical liposomal IFN alpha (F#10-80) for 24 h.

**DOSE REGIMEN I:**

| Condyloma lesion # | Size $\phi$ (mm) | Weight | Surface area (cm$^2$) | IFN applied (IU) | IFN in WHOLE lesion (IU) | IFN in deeper layers (IU) |
|---|---|---|---|---|---|---|
| 1 | 5 | 0.0794 | 0.78 | 560,000 | 29,800 | 25,700 |
| 2 | 4 | 0.0509 | 0.50 | 496,000 | 31,500 | 29,200 |
| 3 | 7 | 0.0542 | 1.54 | 864,000 | 18,100 | 13,100 |
| 4 | 4.5 | 0.0788 | 0.64 | 432,000 | 70,700 | 57,800 |
| 5 | 5 | 0.0807 | 0.78 | 424,000 | 31,000 | 25,300 |
| 6 | 7 | 0.0621 | 1.54 | 240,000 | 17,400 | 13,900 |
| 7 | 6 | 0.0766 | 1.13 | 672,000 | 42,100 | 37,700 |
| Average | 5.5 | 0.0689 | 0.95 | 527,000 | 34,400±18,100 (6.5% of total applied) | 29,000±15,300 (5.5% of total applied) |

**DOSE REGIMEN II:**

| Condyloma lesion # | Size $\phi$ (mm) | Weight | Surface area (cm$^2$) | IFN applied (IU) | IFN in WHOLE lesion (IU) | IFN in deeper layers (IU) |
|---|---|---|---|---|---|---|
| 8 | 6 | 0.0925 | 1.13 | $1.34 \times 10^6$ | 65,300 | 56,700 |
| 9 | 8 | 0.1235 | 2.01 | $2.82 \times 10^6$ | 525,100 | 464,700 |
| 10 | 12 | 0.1845 | 4.52 | $2.42 \times 10^6$ | 380,200 | 338,200 |
| Average | 8.7 | 0.1335 | 2.55 | $2.19 \times 10^6$ | 323,500±235,100 (14.8% of total applied) | 286,500±208,900 (9.5% of total applied) |

## TABLE 15

### Formulation of topical liposomal IFN products.

| FORMULA | | | METHOD OF | ENCAPSULATION |
|---|---|---|---|---|
| NO. | Lipid phase mg/g product | Aqueous phase µl/g product | MANUFACTURE | EFFICIENCY* |
| 1 | Phospholipon 90H 70<br>Cholesterol 18<br>Stearic acid 18<br>Propylene glycol 70 | 10.48 IFN stock<br><br>$(20 \times 10^6 \text{ IU})$<br>$H_2O$ q.s. | Fusion | $55.9\pm1.5\%$ |
| 2 | Phospholipon 90H 70<br>Cholesterol 18<br>Stearic acid 18<br>Propylene glycol 70 | 10.48 IFN stock<br><br>$(20 \times 10^6 \text{ IU})$<br>$H_2O$ q.s. | Solvent<br>evaporation | 57.8% |
| 3 | Phospholipon 90H 100<br>Cholesterol 18<br>Oleic acid 6<br>Stearic acid 6<br>Erucic acid 6<br>Ascorbyl palmitate 0.5<br>Propylene glycol 70 | 10.48 IFN stock<br><br>$(20 \times 10^6 \text{ IU})$<br>$H_2O$ q.s. | Fusion | 57.3% |
| 4 | Phospholipon 90 200<br>Ascorbyl palmitate 1 | 5.24 IFN stock<br>$H_2O$ q.s. | Fusion | |
| 5 | Phospholipon 90H 100<br>Cholesterol 20<br>Stearic acid 10<br>Bovine brain extract<br>Type VIII 5<br>Propylene glycol 70 | 10.48 IFN stock<br><br>$(20 \times 10^6 \text{ IU})$<br>$H_2O$ q.s. | Fusion | 59.2% |
| 6 | Phospholipon 90H 50<br>Phospholipon 90 50<br>Cholesterol 20<br>Bovine brain extract<br>Type III 10<br>Propylene glycol 70 | 10.48 IFN stock<br><br>$(20 \times 10^6 \text{ IU})$<br>$H_2O$ q.s. | Fusion | $69.8\pm2.3\%$ |

EP 0 711 148 B1

TABLE 15 (cont'd)

| FORMULA | | | METHOD OF | ENCAPSULATION |
|---|---|---|---|---|
| NO. | Lipid phase mg/g product | Aqueous phase µl/g product | MANUFACTURE | EFFICIENCY* |
| 7 | Phospholipon 90H 50<br>Phospholipon 90 50<br>Cholesterol 20<br>Stearylamine 10<br>Propylene glycol 70 | 10.48 IFN stock<br><br>(20 x $10^6$ IU)<br>PBS q.s. | Fusion | 65.9% |
| 8 | Phospholipon 90H 50<br>Phospholipon 90 50<br>COMPOUND A 20<br>Cholesterol 20<br>Propylene glycol 70 | 10.48 IFN stock<br><br>(20 x $10^6$ IU)<br>PBS q.s. | Fusion | 79.0% |
| 9 | Phospholipon 90H 50<br>Phospholipon 90 50<br>COMPOUND A 10<br>Cholesterol 20<br>Propylene glycol 70 | 10.48 IFN stock<br><br>(20 x $10^6$ IU)<br>PBS q.s. | Fusion | 77.8±1.3% |
| 10 | Phospholipon 90H 100<br>COMPOUND B 20<br>Cholesterol 20<br>Propylene glycol 70 | 10.48 IFN stock<br><br>(20 x $10^6$ IU)<br>PBS q.s. | Fusion | 47.0±1.3% |
| 11 | Phospholipon 90H 100<br>COMPOUND A 10<br>COMPOUND B 10<br>Cholesterol 20<br>Propylene glycol 70 | 10.48 IFN stock<br><br>(20 x $10^6$ IU)<br>PBS q.s. | Fusion | 75.2% |
| 12 | Phospholipon 90H 50<br>Phospholipon 90 50<br>COMPOUND B 10<br>Cholesterol 20<br>Propylene glycol 70 | 10.48 IFN stock<br><br>(20 x $10^6$ IU) ·<br>PBS q.s. | Fusion | 45.7% |
| 13 | Phospholipon 90 100<br>COMPOUND A 20<br>Cholesterol 20<br>Propylene glycol 70 | 10.48 IFN stock<br><br>(20 x $10^6$ IU)<br>PBS q.s. | Fusion | 73.1% |

EP 0 711 148 B1

38

TABLE 15 (cont'd)

| FORMULA | | | METHOD OF | ENCAPSULATION |
|---|---|---|---|---|
| NO. | Lipid phase mg/g product | Aqueous phase µl/g product | MANUFACTURE | EFFICIENCY* |
| 14 | Phospholipon 90H 100<br>Centrolex P 10<br>Cholesterol 20<br>Propylene glycol 70 | 10.48 IFN stock<br><br>(20 x $10^6$ IU)<br>PBS q.s. | Fusion | 54.8% |

* encapsulation efficiency measurements are the average of 2-5 separate experiments

COMPOUND A = dipalmitoyllysine

COMPOUND B = monolauroyllysine

## TABLE 16

### Properties of liposomes containing IFN alpha.

| Formula No. | Organoleptic properties | Stability* | Microscopy/liposome size distribution |
|---|---|---|---|
| 1 | white cream, homogeneous | (7) | MLV, 1-30μm, aggregation, fusion |
| 2 | white cream, homogeneous | (7) | MLV, 1-10μm, even distribution of liposomes, no fusion or aggregation |
| 3 | white lotion, homogeneous | (7) | MLV, 1-10μm, even distribution of liposomes, no aggregation or fusion |
| 4 | yellow liquid, not appealing | | no liposomes formed |
| 5 | white soft cream, homogeneous | (5) | MLV, 1-15μm, even distribution of liposomes, some fusion, no aggregation |
| 6 | yellowish-wine lotion, homogeneous | (5) | MLV, 1-5μm, even distribution of liposomes, some fusion, no aggregation |
| 7 | yellowish-white cream, homogeneous | (5) | MLV, 1-15μm, signs of phase separation, no aggregation or fusion |
| 8 | white cream, homogeneous | (8) | MLV, 1-5μm, even distribution of liposomes, no fusion or aggregation |
| 9 | white viscous cream, homogeneous | (8) | MLV, 1-5μm, even distribution of liposomes, no fusion or aggregation |
| 10 | white liquid, not homogeneous | (1) | MLV, 1-20μm, aggregation, amorphous particles are present |
| 11 | white soft cream, homogeneous | (6) | MLV, 1-10μm, signs of phase separation, some fusion, no aggregation, few amorphous particles are present |
| 12 | white liquid, homogeneous | (6) | MLV, 1-5μm, signs of phase separation, some fusion, no aggregation, amorphous particles are present |
| 13 | white lotion, not homogeneous | (6) | Liposomes? or emulsion?, amorphous particles and membrane fragments are present |
| 14 | white lotion, homogeneous | (5) | MLV, 1-20μm, aggregation, fusion, signs of phase separation |

* Stability of liposomes was evaluated after 1 month according to the following rating scale:

EP 0 711 148 B1

## TABLE 16 (cont'd)

Visual stability rating for disperse system products (S.A. Hanna, 1989)

9 No visual separation, completely homogeneous

8 No visual separation, virtually homogeneous

7 Very indistinct separation, no clear layer at bottom or top

6 Indistinct separation, no clear layer at bottom or top

5 Distinct separation, no clear layer at bottom or top

4 Homogeneous top or bottom layer, clear layer at bottom or top

3 Distinct separation, clear layer at bottom or top with no coalescence

2 Distinct separation with slight coalescence

1 Distinct separation with slight coalescence

0 Complete separation and complete coalescence

## Claims

1. A liposomal composition for topical administration of an agent having a pharmaceutical, pharmacological or cosmetic effect, comprising

a suspension of multilamellar liposomes obtainable by (i) preparing an oil-in-water emulsion, said oil-in-water emulsion stabilized by a surfactant and (ii) mixing said oil-in-water emulsion with vesicle-forming lipids, said liposomes being composed of (i) a lipid-bilayer outer membrane composed of said vesicle-forming lipids, (ii) a central core compartment containing said oil-in-water emulsion and (iii) an entrapped agent having a pharmaceutical, pharmacological or cosmetic effect.

2. The composition according to claim 1, wherein said oil-in-water emulsion further comprises a lipophilic compound selected from the group consisting of fatty alcohols, waxes, fatty alcohol esters, fatty acid esters, glyceride esters, white petrolatum and mixtures thereof.

3. The composition according to claim 1 or 2, wherein said oil-in-water emulsion is stabilized by a cationic surfactant.

4. The composition according to claim 3, wherein said cationic surfactant is selected from the group consisting of linoleamidopropyl PG-dimonium chloride phosphate, cocamidopropyl PG-dimonium chloride phosphate and stearamidopropyl PG-dimonium chloride phosphate.

5. The composition according to any of the preceding claims, wherein said lipid-bilayer outer membrane further comprises a lipid selected from steroids, saturated fatty acids, unsaturated fatty acids, fatty amines and fatty acylated proteins and peptides.

6. The composition according to any of the preceding claims, wherein said liposome suspension further includes a penetration enhancer.

7. The composition according to claim 6, wherein said penetration enhancer is methyl salicylate or a fatty acylated amino acid.

8. The composition according to claim 7, wherein said penetration enhancer is mono-lauroyllysine.

9. The composition according to any of the preceding claims, wherein said entrapped agent is selected from prostaglandins, local anaesthetic agents, proteins, peptides, antiviral agents, vitamins, antiinflammatory agents, antifungal agents, corticosteroids, plant extracts, amino acids and nucleoside analogues.

10. The composition according to any of claims 1-8, wherein said entrapped agent is selected from prostaglandin $E_1$, tetracaine, lidocaine, etidocaine, interferon alpha, interferon gamma, adenine-$\beta$-D-arabinofuranoside (Ara-A), 5-methoxymethyl-2'-deoxyuridine (MMUdR), vitamin A, vitamin C, vitamin E, provitamin $B_5$, azulene, allantoin, alphabisabolol and L-carnitine.

11. The composition according to any of the preceding claims, wherein said preparing includes blending a lipophilic substance in an aqueous medium in the presence of said surfactant to form said oil-in-water emulsion, and said mixing includes adding said oil-in-water emulsion to vesicle-forming lipids solubilized in a pharmaceutically acceptable hydrophilic solvent other than water.

12. Use of prostaglandin $E_1$ as entrapped agent for the preparation of a liposomal composition according to any of claims 1-11, for use in treatment of impotence.

13. Use of interferon alpha as entrapped agent for the preparation of a liposomal composition according to any of claims 1-11, for use in treatment of an infection caused by human papillomavirus.

14. A process for the preparation of a liposomal composition according to any one of claims 1 to 11 which comprises preparing an oil-in-water emulsion, said oil-in-water emulsion being stabilized by a surfactant and mixing said oil-in-water emulsion with vesicle forming lipids.

**Patentansprüche**

1. Liposomale Zusammensetzung zur topischen Verabreichung eines Agens, das eine pharmazeutische, pharmakologische oder kosmetische Wirkung aufweist, umfassend eine Suspension von multilamellaren Liposomen, die erhältlich sind durch (i) die Herstellung einer Öl-in-Wasser-Emulsion, wobei die Öl-in-Wasser-Emulsion durch eine oberflächenaktive Substanz stabilisiert ist, und (ii) das Mischen der Öl-in-Wasser-Emulsion mit vesikelformenden Lipiden, wobei die Liposomen aus (I) einer äußeren Lipid-Doppelschicht-Membran, die aus den vesikelformenden Lipiden aufgebaut ist, (II) einem zentralen Kernbereich, der die Öl-in-Wasser-Emulsion enthält, und (III) einem darin enthaltenen Agens, das eine pharmazeutische, pharmakologische oder kosmetische Wirkung aufweist, zusammengesetzt sind.

**2.** Zusammensetzung nach Anspruch 1, wobei die Öl-in-Wasser-Emulsion weiterhin eine lipophile Verbindung umfaßt, die ausgewählt ist aus der Gruppe bestehend aus Fettalkoholen, Wachsen, Fettalkoholestern, Fettsäureestern, Glycerinestern, weißem Petrolatum und Gemischen davon.

**3.** Zusammensetzung nach Anspruch 1 oder 2, wobei die Öl-in-Wasser-Emulsion durch eine kationische oberflächenaktive Substanz stabilisiert ist.

**4.** Zusammensetzung nach Anspruch 3, wobei die kationische oberflächenaktive Substanz ausgewählt ist aus der Gruppe bestehend aus Linolamidpropyl-PG-Dimoniumchloridphosphat, Cocamidpropyl-PG-Dimoniumchloridphosphat und Stearamidpropyl-PG-Dimoniumchloridphosphat.

**5.** Zusammensetzung nach einem der vorangegangenen Ansprüche, wobei die äußere Lipid-Doppelschicht-Membran weiterhin ein Lipid umfaßt, ausgewählt aus Steroiden, gesättigten Fettsäuren, ungesättigten Fettsäuren, Fettaminen und fettacylierten Proteinen und Peptiden.

**6.** Zusammensetzung nach einem der vorangegangenen Ansprüche, wobei die Liposom-Suspension weiterhin eine penetrationsverstärkende Substanz beinhaltet.

**7.** Zusammensetzung nach Anspruch 6, wobei die penetrationsverstärkende Substanz Methylsalicylat oder eine fettacylierte Aminosäure ist.

**8.** Zusammensetzung nach Anspruch 7, wobei die penetrationsverstärkende Substanz Monolauroyllysin ist.

**9.** Zusammensetzung nach einem der vorangegangenen Ansprüche, wobei das darin enthaltene Agens ausgewählt ist aus Prostaglandinen, Lokalanästhetika, Proteinen, Peptiden, antiviralen Agenzien, Vitaminen, entzündungshemmenden Agenzien, antimykotischen Agenzien, Corticosteroiden, Pflanzenextrakten, Aminosäuren und Nucleosidanaloga.

**10.** Zusammensetzung nach einem der Ansprüche 1 bis 8, wobei das darin enthaltene Agens ausgewählt ist aus der Gruppe bestehend aus Prostaglandin $E_1$, Tetracain, Lidocain, Etidocain, Interferon-alpha, Interferon-gamma, Adenin-β-D-Arabinofuranosid (Ara-A), 5-Methoxy-methyl-2'-desoxyuridin (MMUdR), Vitamin A, Vitamin C, Vitamin E, Provitamin $B_5$, Azulen, Allantoin, alpha-Bisabolol und L-Carnitin.

**11.** Zusammensetzung nach einem der vorangegangenen Ansprüche, wobei die Herstellung das Mischen einer lipophilen Substanz in einem wässrigen Medium in Gegenwart der oberflächenaktiven Substanz beinhaltet, um die Öl-in-Wasser-Emulsion zu erzeugen, und das Mischen das Zugeben der Öl-in-Wasser-Emulsion zu den vesikelformenden Lipiden beinhaltet, die in einem pharmazeutisch verträglichen hydrophilen Lösungsmittel, das kein Wasser ist, solubilisiert sind.

**12.** Verwendung von Prostaglandin $E_1$ als das darin enthaltene Agens zur Herstellung einer liposomalen Zusammensetzung nach einem der Ansprüche 1 bis 11 zur Verwendung bei der Behandlung von Impotenz.

**13.** Verwendung von Interferon-alpha als das darin enthaltene Agens zur Herstellung einer liposomalen Zusammensetzung nach einem der Ansprüche 1 bis 11 zur Verwendung bei der Behandlung einer Infektion, die durch humanes Papillomavirus verursacht ist.

**14.** Verfahren zur Herstellung einer liposomalen Zusammensetzung nach einem der Ansprüche 1 bis 11, das die Herstellung einer Öl-in-Wasser-Emulsion, wobei die Öl-in-Wasser-Emulsion durch eine oberflächenaktive Substanz stabilisiert ist, und das Mischen der Öl-in-Wasser-Emulsion mit vesikelformenden Lipiden umfaßt.

**Revendications**

**1.** Composition liposomale pour une administration topique d'un agent ayant un effet pharmaceutique, pharmacologique ou cosmétique, comprenant

une suspension de liposomes multilamellaires pouvant être obtenue en (i) préparant une émulsion huile-dans-eau, ladite émulsion huile-dans-eau étant stabilisée à l'aide d'un tensio-actif et (ii) en mélangeant ladite émulsion huile-dans-eau avec des lipides formant des vésicules, lesdits liposomes étant composés (i) d'une membrane externe à bicouche lipidique composée desdits lipides formant des vésicules, (ii) d'un compartiment de noyau central contenant ladite émulsion huile-dans-eau et (iii) d'un agent piégé ayant un effet pharmaceutique, pharmacologique ou cosmétique.

2. Composition selon la revendication 1, dans laquelle ladite émulsion huile-dans-eau comprend en outre un composé lipophile choisi dans le groupe formé par les alcools gras, les cires, les esters d'alcools gras, les esters d'acides gras, les esters de glycérides, la vaseline pure et leurs mélanges.

3. Composition selon la revendication 1 ou 2, dans laquelle ladite émulsion huile-dans-eau est stabilisée par un tensio-actif cationique.

4. Composition selon la revendication 3, dans laquelle ledit tensio-actif cationique est choisi dans le groupe formé par le phosphate de chlorure de linoléamidopropyl-PG-dimonium, le phosphate de chlorure de coco-amidopropyl-PG-dimonium et le phosphate de chlorure de stéaramidopropyl-PG-dimonium.

5. Composition selon l'une quelconque des revendications précédentes, dans laquelle ladite membrane externe à bicouche lipidique comprend en outre un lipide choisi parmi les stéroïdes, les acides gras saturés, les acides gras insaturés, les amines grasses et les protéines et peptides gras acylés.

6. Composition selon l'une quelconque des revendications précédentes, dans laquelle ladite suspension de liposomes comprend en outre un agent favorisant la pénétration.

7. Composition selon la revendication 6, dans laquelle ledit agent favorisant la pénétration est un salicylate de méthyle ou un acide aminé acylé gras.

8. Composition selon la revendication 7, dans laquelle ledit agent favorisant la pénétration est la monolauryl-lysine.

9. Composition selon l'une quelconque des revendications précédentes, dans laquelle ledit agent piégé est choisi parmi les prostaglandines, les agents anesthésiques locaux, les protéines, les peptides, les agents anti-viraux, les vitamines, les agents anti-inflammatoires, les agents antifongiques, les corticostéroïdes, les extraits végétaux, les acides aminés et les analogues de nucléosides.

10. Composition selon l'une quelconque des revendications 1 à 8, dans laquelle ledit agent piégé est choisi parmi la prostaglandine $E_1$, la tétracaïne, la lidocaïne, l'étidocaïne, l'interféron alpha, l'interféron gamma, l'adénine-$\beta$-D-arabinofuranoside (Ara-A), la 5-méthoxy-méthyl-2'-désoxyuridine (MMUdR), la vitamine A, la vitamine C, la vitamine E, la provitamine $B_5$, l'azulène, l'allantoïne, l'alpha-bisabolol et la L-carnitine.

11. Composition selon l'une quelconque des revendications précédentes, dans laquelle ladite préparation consiste à mélanger une substance lipophile dans un milieu aqueux en présence dudit tensio-actif afin de former ladite émulsion huile-dans-eau, et ledit mélange consiste à ajouter ladite émulsion huile-dans-eau à des lipides formant des vésicules, solubilisés dans un solvant hydrophile acceptable sur le plan pharmaceutique, autre que l'eau.

12. Utilisation de la prostaglandine $E_1$ en tant qu'agent piégé pour la préparation d'une composition liposomale selon l'une quelconque des revendications 1 à 11, en vue d'une utilisation dans le traitement de l'impuissance.

13. Utilisation de l'interféron alpha en tant qu'agent piégé pour la préparation d'une composition liposomale selon l'une quelconque des revendications 1 à 11, en vue d'une utilisation dans le traitement d'une infection provoquée par un virus de papillome humain.

14. Procédé pour la préparation d'une composition liposomale selon l'une quelconque des revendications 1 à 11 comprenant la préparation d'une émulsion huile-dans-eau, ladite émulsion huile-dans-eau étant stabilisée par un tensio-actif et le mélange de ladite émulsion huile-dans-eau avec des lipides formant des vésicules.

FIG.Ia

FIG.Ib

FIG. 2

FIG. 3

FIG. 4